# EUROPEAN PATENT APPLICATION

(11) **EP 4 212 544 A1**
(43) Date of publication of application: **19.07.2023**
(21) Application number: 21864487.0
(22) Date of filing: 29.01.2021
(51) Int. Cl.: C07K 14/005, A61K 39/215, A61P 31/14

(54) **CORONAVIRUS-DERIVED RECEPTOR BINDING DOMAIN VARIANT WITH REDUCED ACE2 BINDING CAPACITY, AND VACCINE COMPOSITION COMPRISING SAME**

(30) Priority: 07.09.2020 KR 20200113989
(71) Applicant: GI Cell, Inc., Seongnam-si, Gyeonggi-do 13201 (KR)
(72) Inventor: JANG, Myoung Ho, Seoul 05849 (KR); PARK, Jae Chan, Seoul 05853 (KR); CHOI, Young Joo, Hwaseong-si Gyeonggi-do 18479 (KR); PARK, Young Hyun, Seoul 08365 (KR); KIM, Gil-Jung, Seongnam-si Gyeonggi-do 13506 (KR); JEONG, Seung Mi, Seongnam-si, Gyeonggi-do 13362 (KR); PARK, Yeon Soo, Seoul 04422 (KR); LEE, Su Bin, Daejeon 34232 (KR)
(74) Representative: Mewburn Ellis LLP
(86) International application number: PCT/KR2021/001246
(87) International publication number: WO 2022/050521

(57) **Abstract**

Disclosed are a novel coronavirus-derived receptor-binding domain variant having reduced ACE2-binding affinity, a fusion protein comprising the same, and the use thereof. It is possible to overcome the drawbacks of conventional vaccines using the coronavirus spike protein or receptor-binding domain thereof, wherein the reduced ACE2 expression due to binding to ACE2 and negative feedback may lead to side effects of the lungs or heart, and in particular, may be fatal to patients suffering from underlying diseases of the lungs or heart. In particular, the fusion protein constructed by fusing the coronavirus receptor-binding domain with the Fc domain is imparted with a greatly improved in-vivo half-life, and has superior efficacy by further combining N protein, M protein, ORF protein, or the like of SARS-CoV-2 therewith through additional modification and thus is highly applicable to a multivalent immunogenic composition. Therefore, the coronavirus receptor-binding domain variant is useful for the prevention and treatment of coronavirus infections comprising SARS-CoV-2.

## Description

### BACKGROUND OF THE INVENTION

### Field of the Invention

The present invention relates to a novel coronavirus-derived receptor-binding domain variant having reduced ACE2-binding affinity, a fusion protein comprising the same, and the use thereof.

### Description of the Related Art

Coronavirus is an RNA virus that contains genetic information in the form of ribonucleic acids (RNA). Coronavirus causes respiratory and digestive system infections in humans and animals. In general, coronavirus is readily infected by mucosal transmission, droplet transmission and the like, and humans usually exhibit mild respiratory infections, but occasionally infection is fatal.

Severe acute respiratory syndrome-coronavirus-2 (SARS-CoV-2) arose in Wuhan, China and spread rapidly around the world. The World Health Organization (WHO) gave the name "COVID-19" to the disease caused by the virus. In accordance with the spread of SARS-CoV-2 infection, the WHO declared a "Public Health Emergency of International Concern" (PHEIC) on January 30. Because the number of confirmed COVID-19 cases continued to increase all over the world, the WHO declared a global pandemic for the third time in history, following the Hong Kong Flu on March 11 (1968) and the Swine Flu (2009).

About 100 million people have been infected worldwide, about 2 million people have died, tens of thousands to hundreds of thousands of confirmed patients are added every day, and the number of infected patients continues to increase (As of January 18, 2021, COVID-19 Dashboard by the Center for Systems Science and Engineering (CSSE) at Johns Hopkins University (JHU)). In Korea, about 70,000 patients were confirmed, and hundreds of confirmed cases are added every day due to the spread by infection through local communities, and a total of 1,264 deaths have been reported (as of January 18, 2021, Central Disaster and Safety Countermeasures Headquarters in Korea).

Infection with SARS-CoV-2 is known to cause fever (83-99%), cough (59-82%), loss of appetite (40-84%), fatigue (44-70%), dyspnea (31-40%), phlegm and cough (28-33%), muscle pain and other pain (11-35%) and the like (US Centers for Disease Control and Prevention (CDC). 6 April 2020), and these symptoms were reported to develop after an incubation period of 2 to 14 days.

SARS-CoV-2 is highly contagious, is known to be generally transmitted through direct/indirect contact or aerosol-type droplets discharged from infected persons, and is also reported to often be transmitted through asymptomatic patients (European Centre for Disease Prevention and Control. Retrieved 30 April 2020). In addition, cases of suffering from sequelae or reinfection even after complete recovery have been repeatedly reported. Therefore, the prevention of SARS-CoV-2 infection is of utmost importance. For this purpose, masks, hand washing and social distancing are actively performed at the personal/social levels, but cases of SARS-CoV-2 infection occur at various sites comprising churches, and the infection route thereof is unclear. Thus, there is urgent demand for the development of SARS-CoV-2 vaccines. Vaccine candidates are currently being developed and researched by various institutions, and various types of vaccines such as recombinant vaccines, inactive viral vaccines and mRNA vaccines are in clinical trials. Recently, vaccines developed by some pharmaceutical manufacturers such as Pfizer, Moderna, and Astrazeneca have been urgently approved and released, but production and supply are extremely insufficient to meet the demand, and moreover, multiple variants of coronavirus are sequentially arising. Accordingly, there is continuous need for the development of novel vaccines.

Meanwhile, angiotensin-converting enzyme 2 (ACE2) is known to protect the kidneys, lungs and heart from inflammation by degrading angiotensin II, which is a pro-inflammatory substance. ACE2 is known to play an important role as a viral receptor in the cell entry process of the coronavirus family such as SARS-CoV-1 and SARS-CoV-2. Specifically, SARS-CoV-2 has been reported to act through two mechanisms, namely direct fusion due to co-action with TMPRSS2 and intracellular action based on the binding of ACE 2 and the spike protein (S protein) (Int. J. Mol. Sci. 2020, 21, 5224; 10.1016/j.cell.2020.02.052 et al.). Therefore, strategies for developing many therapeutic agents and vaccines that are currently being researched target spike proteins, and in particular, most vaccines aim to form neutralizing antibodies against the spike proteins of SARS-CoV-2.

Most recombinant vaccines that are introduced only with the antigenic determinant site of the pathogen separately produced using the genetic information of the pathogen have an antibody-mediated virus-neutralizing effect through the formation of a neutralizing antibody against spike protein or RBD as a pharmacological mechanism by administering spike proteins or fragments thereof, particularly, the receptor-binding domain (RBD) which bind to ACE2, as an antigen.

In particular, the receptor-binding domain (RBD) is an amino acid of a specific sequence that binds to ACE2, which is comprised in the spike protein (S protein), and the wild-type amino acid sequence (SEQ ID NO: 1) of SARS-CoV-2 and the results of research on the binding structure thereof have been continuously reported (Nature. 30 March; Science. 3 April; Cell. 9 April).

However, previous research on SARS-CoV-1, which has a cell entry mechanism through binding with ACE2, like SARS-CoV-2, showed that, when the spike protein of SARS-CoV-1 binds to ACE2, the expression of ACE2, which performs a protective function in acute lung through negative feedback is inhibited, and thus lung disease is worsened (Keiji et al, NATURE MEDICINE, 2005). In addition, death from SARS-CoV-2 is reported to be mainly due to heart failure and acute kidney injury, and ACE2 blockers have recently been found to have an effect of preventing acute lung damage caused by SARS-CoV. Based thereon, clinical trials of ACE2 blockers as therapeutic agents for COVID-19 are in progress. Therefore, it is understood that the interaction between spike protein and ACE2 affects the expression and exacerbation of disease in the lungs or heart by decreased ACE2 expression, in addition to entry of coronavirus into cells (infection) (Grifoni et al, Cell, 2020).

Based on these reports, administration of a vaccine using the spike protein or receptor-binding domain of SARS-CoV-2 as an immunogen to the human body induces the formation of the SARS-CoV-2 neutralizing antibody, and causes binding of the spike protein or the receptor-binding domain to ACE2, and may result in side effects such as acute injury and failure of the lungs and heart through pathological mechanisms such as decreased expression of ACE2, as described above. Disadvantageously, these side effects may lead to reduced ACE2 expression and fatal side effects, particularly when a vaccine using the spike protein or receptor-binding domain of SARS-CoV-2 as an immunogen is administered to a patient with underlying lung or heart disease.

Under this background art, as a result of extensive efforts to develop vaccines for preventing or treating coronavirus infections that can overcome the side effects in the lungs and heart caused by binding of the coronavirus-derived spike protein receptor-binding domain to ACE2, the present inventors found that a variant having substitution of one or more amino acid sequences of the receptor-binding domain exhibits lower affinity than the wild-type receptor-binding domain and avoids a decrease in the expression of ACE2, and the fusion protein comprising the variant and the Fc protein and/or other coronavirus-derived domain, when used as an immunogen in mouse and monkey animal models, exhibits a remarkably high titer of the receptor-binding domain specific neutralizing antibody formation and high coronavirus neutralizing ability compared to a fusion protein comprising a wild-type receptor-binding domain. Based on this finding, the present invention has been completed.

The information disclosed in this Background section is provided only for better understanding of the background of the present invention, and therefore it may not comprise information that forms the prior art that is already obvious to those skilled in the art.

### SUMMARY OF THE INVENTION

Therefore, the present invention has been made in view of the above problems, and it is one object of the present invention to provide a coronavirus-derived receptor-binding domain variant having reduced ACE2-binding affinity and the use thereof.

It is another object of the present invention to provide a fusion protein comprising the receptor-binding domain variant and the use thereof.

It is another object of the present invention to provide a monovalent or multivalent immunogenic composition comprising the receptor-binding domain variant or the fusion protein comprising the same as an active ingredient.

It is another object of the present invention to provide a composition for preventing or treating coronavirus.

It is another object of the present invention to provide a vaccination method for preventing or treating coronavirus infection.

It is another object of the present invention to provide a method for preventing or treating coronavirus infection.

It is another object of the present invention to provide a nucleic acid encoding the receptor-binding domain variant or the fusion protein comprising the same.

It is another object of the present invention to provide a recombinant vector comprising the nucleic acid.

It is another object of the present invention to provide a host cell into which the nucleic acid or recombinant vector is introduced.

It is another object of the present invention to provide a method for producing the receptor-binding domain variant or the fusion protein comprising the same.

In accordance with one aspect of the present invention, the above and other objects can be accomplished by the provision of a coronavirus-derived receptor-binding domain (RBD) variant having reduced binding affinity to an ACE2 receptor.

In accordance with another aspect, the present invention provides a fusion protein comprising the receptor-binding domain variant.

In accordance with another aspect, the present invention provides a vaccine composition comprising the receptor-binding domain (RBD) variant and/or the fusion protein.

In accordance with another aspect, the present invention provides the use of the receptor-binding domain (RBD) variant and/or the fusion protein for the manufacture of a vaccine composition.

In accordance with another aspect, the present invention provides a method for vaccination against coronavirus infection, the method comprising administering the receptor-binding domain (RBD) variant, the fusion protein and/or the vaccine composition to a subject.

In accordance with another aspect, the present invention provides a pharmaceutical composition for preventing or treating coronavirus infection comprising the receptor-binding domain (RBD) variant and/or the fusion protein as an active ingredient.

In accordance with another aspect, the present invention provides the use of the receptor-binding domain (RBD) variant and/or the fusion protein for the manufacture of a pharmaceutical composition for preventing or treating coronavirus infection.

In accordance with another aspect, the present invention provides a method for preventing or treating coronavirus infection, the method comprising administering the receptor-binding domain (RBD) variant, the fusion protein and/or the pharmaceutical composition to a subject.

In accordance with another aspect, the present invention provides the use of the receptor-binding domain (RBD) variant and/or the fusion protein for the prevention or treatment of coronavirus infection.

In accordance with another aspect, the present invention provides a nucleic acid encoding the receptor-binding domain (RBD) variant or the fusion protein.

In accordance with another aspect, the present invention provides a recombinant vector comprising the nucleic acid.

In accordance with another aspect, the present invention provides a host cell into which the nucleic acid or recombinant vector is introduced.

In accordance with another aspect, the present invention provides a method for producing a receptor-binding domain (RBD) variant or a fusion protein comprising the same, the method comprising culturing the host cell.

### BRIEF DESCRIPTION OF THE DRAWINGS

The above and other objects, features and other advantages of the present invention will be more clearly understood from the following detailed description taken in conjunction with the accompanying drawings, in which:
FIG. 1 shows a binding structure between a SARS-CoV-2 receptor-binding domain (RBD) and ACE2 (Nature. 30 March);
FIG. 2 shows the amino acid sequence of SARS-CoV-2-derived wild-type RBD, wherein each shaded part indicates a receptor-binding motif (RBM) and the blue boxes indicate epitopes of MHC I and MHC II. Red amino acids represent the screened mutation sites in Example of the present invention;
FIG. 3 is a schematic diagram showing the fusion protein comprising wild-type RBD or variant RBD prepared in Example;
FIG. 4 is a schematic diagram showing IgGl-Fc (left), and shows the results of SDS-PAGE on the product (right).
FIG. 5 is a schematic diagram showing RBDwt-Fc (left) and shows the results of SDS-PAGE on the product (right);
FIG. 6 is a schematic diagram of Fc-RBDwt (left) and shows the results of SDS-PAGE on the product (right);
FIG. 7 is a schematic diagram showing N-Fc (left) and shows the results of SDS-PAGE on the product (right);
FIG. 8 is a schematic diagram of RBDm(Q175A, N183A)-Fc (left) and shows the results of SDS-PAGE on the product (right);
FIG. 9 is a schematic diagram of RBDm(G164A, V165A, Q175A, S176A, N183A)-Fc (left) and shows the results of SDS-PAGE on the product (right);
FIG. 10 is a schematic diagram of Fc-RBDm(L137A, F168A) (left) and shows the results of SDS-PAGE on the product (right) ;
FIG. 11 is a schematic diagram of RBDm(L137A, F168A)-Fc (left) and shows the results of SDS-PAGE on the product (right);
FIG. 12 is a schematic diagram of RBDm(L137A, G164A, V165A, F168A, Q175A, S176A, N183A)-Fc (left) and shows the results of SDS-PAGE on the product (right);
FIG. 13 is a schematic diagram of GIC-1114 (left) and shows the results of SDS-PAGE on the product (right);
FIG. 14 is a schematic diagram of GIC-1114m(L137A, F168A) (left) and shows the results of SDS-PAGE on the product (right);
FIG. 15 is a schematic diagram of GIC-1114m(L137A) (left) and shows the results of SDS-PAGE on the product (right);
FIG. 16 is a schematic diagram of GIC-1114m(F168A) (left) and shows the results of SDS-PAGE on the product (right);
FIG. 17 is a schematic diagram of GIC-1114m(G184D) (left) and shows the results of SDS-PAGE on the product (right);
FIG. 18 is a schematic diagram of Fc-RBDm(L137A, G164A, V165A, F168A, Q175A, S176A, N183A);
FIG. 19 is a schematic diagram showing GIC-1114N;
FIG. 20 is a schematic diagram showing GIC-1114Nm(L137A, F168A);
FIG. 21 is a schematic diagram showing GIC-1114m(L137A, G164A, V165A, F168A, Q175A, S176A, N183A);
FIG. 22 is a schematic diagram showing GIC-1151;
FIG. 23 is a schematic diagram of GIC-1151m(L137A, F168A) (left) and shows the results of SDS-PAGE on the product (right);
FIG. 24 is a schematic diagram of GIC-1132m(L137A, F168A) (left) and shows the results of SDS-PAGE on the product (right);
FIG. 25 is a schematic diagram of GIC-1133m(L137A, F168A) (left) and shows the results of SDS-PAGE on the product (right);
FIG. 26 is a graph showing the binding affinity of the indicated fusion protein at various concentrations to the ACE2 receptor over time (top of each item) and quantification values of the binding affinity;
FIG. 27 is a graph showing the binding affinity of the indicated fusion protein at a single concentration to the ACE2 receptor over time;
FIG. 28 shows the binding efficiency of the indicated fusion protein to the hACE2 receptor;
FIG. 29 shows hACE2 expression reduction (down-regulation) in HEK293 cells when treated with the indicated fusion protein;
FIG. 30 is a schematic diagram showing vaccination of a mouse animal model with the fusion protein of the present invention;
FIG. 31 shows the titer of RBD-specific neutralizing antibody formation in a mouse animal model vaccinated with either Fusion protein GIC-1114 or GIC-1114m alone or in combination with an adjuvant. Wherein GIC-1114 comprises a wild-type RBD, a Fc domain and a SARS-CoV-2-derived N protein and GIC-1114m comprises a variant RBD(L137A, F168A), a Fc domain and the SARS-CoV-2-derived N protein
FIG. 32 shows titer of the SARS-CoV2 RBD-specific neutralizing antibody formation in serum obtained 4 weeks after inoculating mice with GIC-1151m (L137A, F168A) using AddaVax as an adjuvant;
FIG. 33 schematically shows vaccination of a monkey animal model using the fusion protein of the present invention;
FIG. 34 shows the titer of the SARS-CoV-2 spike protein-specific neutralizing antibody formation in a monkey animal model vaccinated with GIC-1114 or GIC-1114m (L137A, F168A) in combination with an adjuvant (AddaVax), wherein the dotted line shows a significant SARS-CoV-2 spike protein target antibody titer (2880 titer) as a vaccine identified by the USFDA (USFDA, Recommendations for Investigational COVID-19 convalescent plasma);
FIG. 35 shows the result of sVNT confirming the SARS-CoV-2 neutralizing ability of monkey serum collected 4 weeks after vaccination with GIC-1114 or GIC-1114m (L137A, F168A) in combination with an adjuvant (AddaVax).
FIG. 36 shows the result of sVNT confirming the SARS-CoV-2 neutralizing ability of monkey serum collected 8 weeks after vaccination with GIC-1114 or GIC-1114m (L137A, F168A) in combination with an adjuvant (AddaVax).

### DETAILED DESCRIPTION OF THE INVENTION

Hereinafter, the present invention will be described in detail. However, the following detailed description is provided only for illustration of the present invention, and should not be construed as limiting the scope of the present invention. A variety of modifications and alterations are possible without departing from the scope of the claims set forth later and equivalents thereto.

Unless otherwise indicated, nucleic acids and amino acids are written from left to right, in the 5' to 3' direction, and in the N-terminus to C-terminus direction. Numerical ranges listed herein comprise the numbers defining the range, and comprise any integer or non-integer fraction within the defined range.

Unless defined otherwise, all technical and scientific terms used herein have the same meanings as appreciated by those skilled in the field to which the present invention pertains. While any methods and materials similar or equivalent to those described herein may be used in practice to test the present invention, preferred materials and methods are described herein.

SARS-CoV-2, which is a novel coronavirus that was first found in China in December 2019, is a pandemic with about 100 million confirmed cases and millions of deaths all over the world. Despite these circumstances, therapeutic agents and vaccines for SARS-CoV-2 that have proven to be effective are severely lacking, and many researchers and companies are making great efforts to develop therapeutic agents and vaccines therefor.

Most SARS-CoV-2 vaccines currently being developed aim to produce neutralizing antibodies using, as immunogens, spike proteins or fragments thereof, which are reported to play the greatest role in the mechanisms of infection (cell entry) and symptom expression of SARS-CoV-2. However, according to reports on SARS-CoV-1, which has a mechanism very similar to SARS-CoV-2, and recent research on the roles of SARS-CoV-2 and ACE2, when a spike protein binds to ACE2, the expression of ACE2 may be reduced, and reduced ACE2 expression in the lungs or heart may exacerbate a disease (Keiji et al, NATURE MEDICINE, 2005; Grifoni et al, Cell, 2020). Thus, a vaccine composition using a spike protein or a fragment thereof as an immunogen has a high probability of causing side effects in the lungs or heart, and particularly, has a disadvantage of being fatal when administered to a patient with an underlying disease.

In one embodiment of the present invention, in an attempt to overcome the drawbacks of a vaccine using such a spike protein or a fragment thereof as an immunogen, a SARS-CoV-2 receptor-binding domain variant was produced by inducing a mutation in the receptor-binding motif amino acid, based on the analysis of the binding structure between the receptor-binding domain of the spike protein derived from SARS-CoV-2 and ACE2, and the receptor-binding domain variant was found to have lower ability to bind to ACE2 and reduced ACE2 expression due to negative feedback compared to a wild-type receptor-binding domain.

In another embodiment of the present invention, a variety of Fc fusion proteins comprising the receptor-binding domain variant of the present invention were produced, and mammalian animal models (mouse, monkey) were vaccinated therewith. The result showed that Fc fusion proteins exhibited remarkably high neutralizing antibody formation and induced T-cell immune responses, and thus exhibited excellent preventive and therapeutic effects against coronavirus infection.

Thus, in one aspect, the present invention is directed to a coronavirus-derived receptor-binding domain (RBD) variant having reduced binding affinity to an ACE2 receptor.

As used herein, the term "receptor-binding motif (RBM)" is an amino acid sequence comprised in the receptor-binding domain (RBD), and has a site contacting the receptor ACE2. For example, the wild-type amino acid sequence (120-188 amino acids of SEQ ID NO: 1) of the SARS-CoV-2-derived receptor-binding motif has been reported (Nature. 30 March).

A coronavirus spike protein is known to play a very important role not only in cell infection with coronavirus, but also in virus reassembly and release. In particular, the receptor-binding domain is a domain that is directly involved in binding and interaction with ACE2, which is a receptor of the spike protein, and is characterized in that the sequence thereof is highly conserved among coronavirus species.

In the present invention, the coronavirus receptor-binding domain or receptor-binding motif may be the receptor-binding domain or motif of the spike protein of currently reported coronavirus or the receptor-binding domain or motif of the spike protein of variant coronavirus having a gene mutation.

In the present invention, the receptor-binding domain may be derived from SARS-CoV-2, but is not limited thereto.

In the present invention, the receptor-binding domain may be represented by the amino acid sequence of SEQ ID NO: 1, but is not limited thereto. The receptor-binding domain may have a sequence having high homology with the amino acid sequence of SEQ ID NO: 1, for example, a sequence having high homology of 80% or more, 90% or more, preferably 95% or more, and most preferably 99% or more.

The receptor-binding domain according to the present invention is to be interpreted as comprising variants or fragments thereof in which amino acid residues are conservatively substituted at specific amino acid residue positions.

As used herein, the term "conservative substitution" refers to modification comprising substitution of one or more amino acids with amino acids having similar biochemical properties without causing loss of the biological or biochemical function of the corresponding protein.

The term "conservative amino acid substitution" refers to substitution of amino acid residues with other amino acid residues having side chains similar thereto. For example, classes of amino acid residues having similar side chains are well known in the art. These classes comprise amino acids having basic side chains (e.g. lysine, arginine, histidine), amino acids having acidic side chains (e.g. aspartic acid, glutamic acid), amino acids having uncharged polar side chains (e.g. glycine, asparagine, glutamine, serine, threonine, tyrosine, cysteine), amino acids having non-polar side chains (e.g., alanine, valine, leucine, isoleucine, proline, phenylalanine, methionine, tryptophan), amino acids having beta-branched side chains (e.g., threonine, valine, isoleucine), and amino acids having aromatic side chains (e.g., tyrosine, phenylalanine, tryptophan, histidine).

In the present invention, the receptor-binding domain binds to ACE2 based on a receptor-binding motif. Therefore, in the present invention, it is preferred that conservative substitutions occur in portions of the amino acid sequence other than the receptor-binding domain and motif, but the invention is not limited thereto.

As used herein, the term "receptor-binding domain variant" is intended to encompass a mutation, preferably substitution, deletion, insertion or the like, of at least one amino acid residue in the wild-type receptor-binding domain sequence, and cleavage of at least one amino acid residue at the N-terminus or C-terminus. Thus, in the present invention, the receptor-binding domain variant is used in a broad sense comprising "a fragment of a receptor-binding domain variant".

In the present invention, the receptor-binding domain variant may comprise a mutation in the amino acid sequence of the receptor-binding motif.

In the present invention, the receptor-binding domain variant preferably comprises a mutation in at least one amino acid selected from the group consisting of L137, G164, V165, F168, Q175, S176, N183 and G184 in the amino acid sequence of SEQ ID NO: 1, preferably a mutation in at least one selected from the group consisting of L137, F168, and G184, more preferably a mutation in at least one selected from the group consisting of a mutation in amino acids L137 and F168 and a mutation in amino acid G184, and most preferably a mutation in amino acids of L137 and F168, but is not limited thereto.

In the present invention, in the broadest sense, the mutation is intended to comprise all amino acid mutations such as substitution, deletion, and insertion, glycosylation of amino acids, substitution of side chains thereof, and the like.

In the present invention, the mutation may be substitution of an amino acid.

In the present invention, the mutation may be substitution with alanine as well as any amino acid residue or other amino acid residue having a side chain similar to alanine, as long as it is capable of reducing the ability to bind to the ACE2 receptor. For example, the mutation may be substitution with lysine, tyrosine, threonine, aspartic acid, valine, leucine, isoleucine, proline, phenylalanine, methionine, tryptophan, or the like, but is not limited thereto.

In the present invention, the receptor-binding domain variant preferably comprises substitution of at least one amino acid residue selected from L137A, G164A, V165A, F168A, Q175A, S176A, N183A and G184D in the amino acid sequence of SEQ ID NO: 1.

In the present invention, the receptor-binding domain variant more preferably comprises at least one substitution selected from the group consisting of substitution of amino acids L137A and F168A; and substitution of amino acid G184D in the amino acid sequence of SEQ ID NO: 1, and most preferably may comprise substitution of amino acids L137A and F168A, but is not limited thereto.

In the present invention, when the receptor-binding domain has an amino acid sequence different from SEQ ID NO: 1 (for example, a receptor-binding domain of a variant coronavirus having a mutation in a spike protein) or a fragment thereof, an amino acid corresponding to the amino acid described above can be easily derived through alignment and analysis by those skilled in the art. In this case, it is obvious that the receptor-binding domain variant of the present invention may comprise a mutation in at least one of amino acids corresponding to the amino acids described above.

In the present invention, the receptor-binding domain variant may be fused with other components such as polypeptides, proteins or sugars and PEGs for modulation of biological properties or physical/chemical properties.

In the present invention, an expression having the form of a one-letter amino acid residue code together with numbers (n), such as "L137", means the name and type of the amino acid residue at the n^{th} position in each amino acid sequence.

For example, "L137" means that the amino acid residue at 137^{th} position in the amino acid sequence of SEQ ID NO: 1 is asparagine. An amino acid residue name after a number, such as "L137A", means substitution of an amino acid, and "L137A" means that leucine (Leu, L) at 137^{th} position of SEQ ID NO: 1 is substituted with alanine (Ala, A).

In one embodiment of the present invention, a fusion protein was produced by fusing the receptor-binding domain variant with an Fc domain of an immunoglobulin. The fusion protein can exhibit improved biological properties such as prolonged half-life and increased expression level while minimizing the loss of biological activity.

In another aspect, the present invention is directed to a fusion protein comprising the receptor-binding domain (RBD) variant of the present invention.

In the present invention, the fusion protein may further comprise other substances in addition to the receptor-binding domain variant. For example, the fusion protein may comprise an Fc domain, a coronavirus-derived substance (preferably a SARS-CoV-2-derived substance), a linker, an adjuvant, an antigen, an antibody, or the like, but is not limited thereto.

In the present invention, the coronavirus-derived substance means any substance derived from coronavirus, such as a coronavirus-derived protein, nucleic acid (preferably, RNA) or polysaccharide.

In the present invention, the coronavirus-derived substance may be a coronavirus-derived protein or a fragment thereof, more preferably a coronavirus-derived N protein or a M protein, and even more preferably a coronavirus-derived N protein or RNA-binding domain thereof.

In the present invention, the coronavirus-derived substance may comprise two or more substances in addition to the receptor-binding domain variant, and may comprise two or more identical receptor-binding domain variants or two or more other substances.

As used herein, the term "coronavirus" refers to an RNA virus that belongs to the genus *Coronavirinae* (Ninth Report of the International Committee on Taxonomy of Viruses. Elsevier, Oxford. pp. 806-828). The genus *Coronavirinae* is divided into four genera, namely alpha, beta, gamma, and delta coronavirus genera. Examples of coronaviruses that may infect humans comprise SARS-CoV, MERS-CoV, SARS-CoV-2, HCoV-229E, HCoV-OC43, HKU1, HCoV-NL63, and the like, but are not limited thereto.

In one embodiment of the present invention, a protein fused with a receptor-binding domain of SARS-CoV-2-derived spike protein and an RNA-binding domain of nucleocapsid protein was produced, but all coronaviruses have a spike protein in common and enter and proliferate in the host cell through binding of spike protein to ACE2, which is a receptor thereof. In particular, it is obvious that the receptor-binding domain comprised in the S1 subunit of each species is highly conserved, and thus the fusion protein of the present invention is not limited to the SARS-CoV-2-derived receptor-binding domain and the RNA-binding domain.

In the present invention, the coronavirus-derived substance may be derived from SARS-CoV-2. For example, the coronavirus-derived substance may be an N protein, M protein, ORF protein, or the like. Preferably, the fusion protein of the present invention may further comprise an N protein and/or an M protein, but is not limited thereto.

In the present invention, the fusion protein may have a structure in which the receptor-binding domain variant is linked to the coronavirus-derived substance.

In the present invention, more preferably, the fusion protein may have a structure in which the receptor-binding domain variant is linked to the N protein derived from SARS-CoV-2, most preferably to the RNA-binding domain of the N protein.

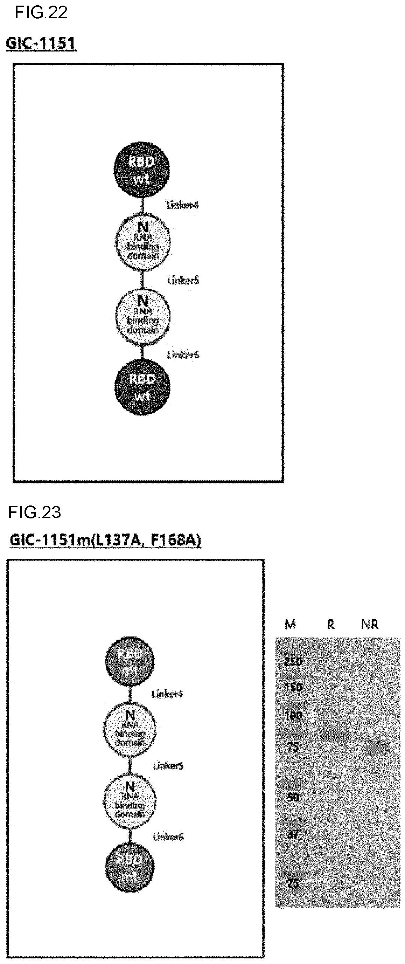

In an embodiment of the present invention, a fusion protein (see FIGS. 22 and 23) in which two receptor-binding domain variants and two N-protein RNA-binding domains are fused by a linker was produced, and the immunogenicity thereof was tested, but the present invention is not limited thereto.

In the present invention, the fusion protein may have a structure in which the receptor-binding domain variant, the N protein (or an RNA-binding domain thereof), the N protein (or an RNA-binding domain thereof), and a receptor-binding domain variant are linked in that order.

In the present invention, the receptor-binding domain variant may be directly linked to at least one of the N protein and the M protein through covalent bonds or the like, and may be indirectly linked thereto via other molecules, for example, linkers, proteins and polysaccharides.

As used herein, the term "linker" refers to a molecule that connects each domain of a fusion protein such as a receptor-binding domain variant, an Fc domain, a coronavirus-derived substance (e.g., an N protein, M protein, etc.) and the like.

In the present invention, the linker may preferably be a glycine-serine linker (GS linker). The glycine-serine linker may be, for example, a G2S, G3S, or G4S linker, but is not limited thereto.

In the present invention, the linker may more preferably comprise an amino acid sequence selected from the group consisting of amino acid sequences of SEQ ID NO: 6 to SEQ ID NO: 11.

In the present invention, the fusion protein may have a sequentially linked structure of a receptor-binding domain variant (RBD) - GS linker - N or M protein, RBD - GS linker - N protein - GS linker - M protein, or RBD - GS linker - M protein - GS linker - N protein, but is not limited thereto.

In the present invention, the fusion protein may have a sequence represented by SEQ ID NO: 60.

In the present invention, the fusion protein may comprise a receptor-binding domain variant and an Fc domain.

As used herein, the term "Fc domain" refers to a tail region of an antibody that interacts with the receptor on the cell surface and with proteins of the complement system in immunoglobulins, comprises the heavy-chain constant domains CH2 and CH3, and may further comprise hinge regions of the heavy-chain constant domains. In the present invention, the Fc domain is intended to encompass all of the Fc domains of an immunoglobulin, a fragment thereof, and a variant thereof.

In one embodiment of the present invention, a fusion protein was produced in the form of a homodimer in which two identical Fc domains to which the receptor-binding domain variant and/or the coronavirus-derived substance are linked through a disulfide bond.

In the present invention, the fusion protein may comprise two or more Fc domains and receptor-binding domain variants.

In the present invention, the fusion protein may be a dimer of a protein comprising a receptor-binding domain variant and an Fc domain.

In the present invention, the fusion protein may be a dimer of a protein comprising a receptor-binding domain variant, an Fc domain, and at least one coronavirus-derived substance.

For example, in the present invention, the dimer may be formed through a disulfide bond of at least one cysteine residue of each Fc domain, but is not limited thereto.

In the present invention, the fusion protein may be a heterodimer or a homodimer of a protein comprising a receptor-binding domain variant and an Fc domain. In an embodiment of the present invention, the fusion protein was prepared as a homodimer, but is not limited thereto.

In the present invention, the Fc domain may be a mammalian immunoglobulin Fc domain, preferably a mouse, rabbit or human immunoglobulin Fc domain, and more preferably a human immunoglobulin Fc domain, but is not limited thereto.

In the present invention, the Fc domain may be an IgA, IgM, IgE, IgD or IgG Fc domain, a fragment thereof, or a variant thereof, and preferably, the Fc domain is an IgG Fc domain (e.g. IgG1, IgG2a, IgG2b, IgG3 or IgG4 Fc domain), but is not limited thereto.

In the present invention, the Fc domain may be a human IgG1 Fc domain. More specifically, the Fc domain may be an Fc domain derived from IgG1, IgG2a, IgG2b, IgG3 or IgG4, which is an IgG isotype derived from an organism.

In the present invention, the Fc domain is most preferably represented by the sequence of SEQ ID NO: 2, or comprises the same, but is not limited thereto.

In addition, the Fc domain may comprise a sugar chain or may take a sugar chain that is increased or decreased compared to the native form, or a form in which a sugar chain is removed, compared to the native form. The increase, decrease, or removal of the sugar chain may be performed by a conventional method known in the art, such as a chemical method, an enzymatic method, and a genetic engineering method using microorganisms. Herein, the removal of the sugar chain from the Fc domain remarkably reduces binding affinity for C1q of the primary complement component, C1, and causes reduction or loss of antibody-dependent cell-mediated cytotoxicity (ADCC) or complement-dependent cytotoxicity (CDC), thus preventing an undesired immune response *in vivo.*

In the present invention, the receptor-binding domain variant may be linked to the N-terminus and/or C-terminus of the Fc domain, and preferably to the C-terminus thereof.

In the present invention, the receptor-binding domain variant may be linked to one of the N-terminus or C-terminus of the Fc domain, and the coronavirus-derived substance may be further linked to a remaining one thereof.

The present invention may be characterized in that the RNA-binding domain of the ORF protein, N protein and/or M protein of the coronavirus, preferably N protein, more preferably RNA-binding domain of N protein is linked to the other terminus of the Fc domain.

In the present invention, components such as the receptor-binding domain variant, the Fc domain and the SARS-CoV-2-derived substance (preferably the N protein and/or M protein), comprised in the fusion protein, are directly linked through covalent bonds or may be linked indirectly through a linker. In an embodiment of the present invention, a fusion protein was produced by linking a receptor-binding domain (or a variant thereof) or SARS-CoV-2-derived N protein to the C-terminus and/or N-terminus of the Fc domain using a linker represented by SEQ ID NO: 6 to SEQ ID NO: 11, but the present invention is not limited thereto.

In the present invention, the fusion protein preferably has a structure in which the receptor-binding domain variant, the Fc domain and the nucleocapsid protein are linked in that order, or in which the nucleocapsid protein, the Fc domain and the receptor-binding domain variant are linked in that order in the sequence, or is a dimer thereof, and the M protein may be further linked to the nucleocapsid protein, but the present invention is not limited thereto.

In the present invention, when the N protein is linked to the other terminus, it acts as an antigen and induces the expression of cytotoxic lymphocytes (CTL) to kill the virus.

As used herein, the term "nucleocapsid protein" refers to a genetic material of a virus and a capsid protein surrounding RNA for coronavirus. Expression of nucleocapsid proteins has been reported to be remarkably increased in coronavirus-infected cells, and in particular, the RNA-binding domain of the nucleocapsid protein is a highly conserved sequence. In the present invention, the term "nucleocapsid protein" is used interchangeably with "N protein", having the same meaning as above.

As used herein, the term "M protein" refers to a membrane protein.

In the present invention, coronavirus-derived substances such as the N protein and M protein are intended to encompass not only the entire protein, but also a fragment of the protein. For example, the N protein is used to mean not only the entire N protein, but also a specific domain or segment of the N protein, and preferably the RNA-binding domain of the N protein, or a fragment comprising the same. The N protein and M protein have high immunogenicity and promote differentiation of cytotoxic lymphocytes.

In the present invention, the N protein may be represented by the sequence of SEQ ID NO: 3, or may comprise the sequence represented by SEQ ID NO: 3.

In the present invention, the fusion protein may comprise the RNA-binding domain of the N protein.

In the present invention, the RNA-binding domain may be represented by SEQ ID NO: 4.

In the present invention, the fusion protein may be represented by any one amino acid sequence selected from the group consisting of SEQ ID NOS: 43 to 48, or may comprise the same.

In the present invention, the fusion protein may be represented by any one amino acid sequence selected from the group consisting of SEQ ID NOS: 50 to 54 and SEQ ID NOS: 56 to 58, or may comprise the same.

In one embodiment of the present invention, it was found that a fusion protein prepared by linking a receptor-binding domain variant to one terminus (the N-terminus or C-terminus) of the Fc domain and linking an RNA-binding domain of an N protein to the other terminus thereof has a high titer of the coronavirus RBD specific-neutralizing antibody formation and T-cell immune response induction ability.

The receptor-binding domain variant and/or the fusion protein of the present invention exhibits immunogenicity and thus can induce various immune responses in the body, and can, for example, induce the formation of neutralizing antibodies, stimulate the differentiation of cytotoxic T lymphocytes, and stimulate the differentiation of Th cells. In one embodiment of the present invention, it was found that the receptor-binding domain variant and/or the fusion protein of the present invention can induce a T-cell immune response comparable or superior to that of a wild-type receptor-binding domain and a fusion protein comprising the same.

Accordingly, in another aspect, the present invention is directed to a vaccine composition comprising the receptor-binding domain variant and/or the fusion protein of the present invention.

As used herein, the term "vaccine composition" refers to a composition comprising a substance that acts as an antigen or immunogen *in vivo* or *in vitro* to induce an immune response, and may be used interchangeably with "vaccine" or "immunogenic composition" having the same meaning as above.

As used herein, the term "immune response" is intended to encompass both innate and adaptive immune responses, for example, complement-mediated immune responses, cell (T cell)-mediated immune responses, and/or antibody (B cell) responses.

The vaccine composition of the present invention may induce or improve an immune response against coronavirus in the subject to which the vaccine composition is administered, and more specifically, may induce formation of neutralizing antibodies against SARS-CoV-2 virus, induce and/or increase differentiation of cytotoxic lymphocytes, prevent, ameliorate, eliminate or reduce the likelihood of reactivation of the virus, and/or prevent or reduce the likelihood of the onset of other diseases or complications associated with reactivation of the virus, when the subject is infected with SARS-CoV-2.

In the present invention, the vaccine composition may further comprises an adjuvant.

As used herein, the term "adjuvant" refers to a concept based on the discovery by Alexander Glenny that an aluminum salt increases the immune response, and refers to an auxiliary component added to induce a stronger immune response in a subject to which an immunogenic composition or vaccine is administered. Examples of the adjuvant may comprise aluminum salts such as aluminum phosphate or aluminum hydroxide, squalene, squalene-containing emulsions such as MF59 or analogues thereof (MF59-like substances), AS03 or analogues thereof (AS03-like substances), AF03 or analogues thereof (AF03-like substances), and SE or analogues thereof (SE-like substances), calcium salts, dsRNA analogues, lipopolysaccharides, lipid A analogues (MPL-A, GLA, etc.), flagellins, imidazoquinolines, CpG ODN, mineral oils, Toll-like receptor (TLR) antagonists, C-type lectin ligands, CD1d ligand (α-galactosylceramide, etc.), detergents, liposomes, saponins such as QS21, cytokines, peptides, and the like, but are not limited thereto.

In one embodiment of the present invention, as an adjuvant, AddaVax (MF59 like), MPLA, Alum or the like was used alone or in combination, and it was confirmed that the immune response was boosted in all cases.

Therefore, in the present invention, the adjuvant is preferably selected from the group consisting of MF59, Alum, MPLA and combinations thereof, but is not limited thereto.

The optimal dosage of the vaccine composition of the present invention can be determined by standard research involving observation of a suitable immune response in a subject. After initial vaccination, the subject may be subjected to one or more booster immunizations at appropriate intervals.

The vaccine composition of the present invention may be administered in a pharmaceutically effective amount, and the term "pharmaceutically effective amount" refers to an amount sufficient to induce or increase an immune response without causing side effects or serious or excessive immune responses. The suitable dosage may vary depending on a variety of factors comprising the formulation method, mode of administration, the age, weight, gender, and pathological condition of the patient, diet, administration time, route of administration, excretion rate and response sensitivity. Various general considerations when determining a pharmaceutically effective amount are known to those of skill in the art, and are set forth in references such as [Gilman et al., eds., Goodman And Gilman's: The Pharmacological Bases of Therapeutics, 8th ed., Pergamon Press, 1990] and [Remington's Pharmaceutical Sciences, 17th ed., Mack Publishing Co., Easton, Pa., 1990].

The vaccine composition of the present invention may be administered in combination with a therapeutic agent for coronavirus or a symptomatic therapeutic agent, and may be administered in combination with other vaccines, virus therapeutic agents, immune adjuvants, symptom relief agents, and the like.

The vaccine composition of the present invention may be prepared into a unit dosage form, or may be incorporated into a multi-dose container by formulating the same using a pharmaceutically acceptable carrier and/or excipient according to a method that can be easily implemented by a person having ordinary skill in the art to which the present invention pertains. The formulation may be prepared and used in the form of oral formulations such as powders, granules, tablets, capsules, suspensions, emulsions, syrups and aerosols, external preparations, suppositories and sterilized injection solutions according to a conventional method. Suitable formulations known in the art may be those disclosed in the reference Remington's Pharmaceutical Science (Mack Publishing Company, Easton PA). Solid formulations for oral administration comprise tablets, pills, powders, granules, capsules and the like. Such solid formulations are prepared by mixing at least one excipient such as starch, calcium carbonate, sucrose, lactose, gelatin and the like. In addition to simple excipients, lubricants such as magnesium stearate and talc are also used. Liquid formulations for oral administration comprise suspensions, oral liquids and solutions, emulsions, syrups and the like. Various excipients such as wetting agents, sweeteners, fragrances, preservatives and the like may be comprised, in addition to water and liquid paraffin, which are simple diluents that are commonly used. Formulations for parenteral administration comprise sterilized aqueous solutions, nonaqueous solutions, suspensions, emulsions, freeze-dried preparations, and suppositories. Examples of a suppository base comprise Witepsol, macrogol, Tween 61, cacao butter, laurin butter, glycerogelatin and the like.

The vaccine composition of the present invention may be administered orally or parenterally. The route of administration of the composition according to the present invention is, for example, intrapulmonary, intravenous, subcutaneous, intramuscular, intraarterial, intramedullary, intrathecal, intracardiac, transdermal, intraperitoneal, intestinal, sublingual, oral or topical administration. The dosage of the composition according to the present invention varies depending on the patent's weight, age, gender, health status, diet, administration time, administration method, excretion rate, or severity of disease, and is easily determined by those skilled in the art. In addition, the composition of the present invention may be prepared into a suitable formulation using known techniques for clinical administration.

In another aspect, the present invention is directed to the use of the receptor-binding domain variant and/or fusion protein for the manufacture of a vaccine composition for preventing coronavirus infection.

In another aspect, the present invention is directed to a method for vaccination against coronavirus infection, the method comprising administering the receptor-binding domain variant, the fusion protein and/or the vaccine composition to a subject.

In an embodiment of the present invention, a receptor-binding domain derived from SARS-CoV-2 was used, but a virus such as SARS-CoV-1 can be also used for the prevention or treatment of all coronavirus infections since it enters cells through a similar mechanism through the binding of ACE2 and spike protein having a conserved sequence (RBM) and is exhibited as a disease.

In another aspect, the present invention is directed to a pharmaceutical composition for preventing or treating coronavirus infection comprising the receptor-binding domain variant and/or the fusion protein as an active ingredient.

In the present invention, the pharmaceutical composition may be a therapeutic vaccine composition for vaccine therapy.

As used herein, the term "prevention" refers to any action causing the suppression or delay of the onset of a disease of interest by administering the pharmaceutical composition according to the present invention.

As used herein, the term "treatment" refers to any action causing an improvement in symptoms of a disease of interest or the beneficial alteration of the symptoms by administering the pharmaceutical composition according to the present invention.

The pharmaceutical composition according to the present invention may further comprise an appropriate carrier, excipient or diluent typically used for pharmaceutical compositions.

Specifically, the carrier, excipient or diluent that may be comprised in the pharmaceutical composition may comprise lactose, dextrose, sucrose, sorbitol, mannitol, xylitol, erythritol, maltitol, starch, acacia rubber, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil. The composition may be formulated using a commonly used diluent or excipient such as a filler, an extender, a binder, a wetting agent, a disintegrant or a surfactant.

The pharmaceutical composition according to the present invention may be used in various formulations according to a conventional method. Suitable formulations comprise oral formulations such as tablets, pills, powders, granules, sugar coated tablets, hard or soft capsules, solutions, suspensions or emulsions, injections and aerosols, external preparations, suppositories and sterile injectable solutions, but are not limited thereto.

The pharmaceutical composition according to the present invention may be prepared in a suitable formulation using a pharmaceutically inert organic or inorganic carrier. That is, when the formulation is a tablet, a coated tablet, a sugar coated tablet, and a hard capsule, it may comprise lactose, sucrose, starch or a derivative thereof, talc, calcium carbonate, gelatin, stearic acid, or a salt thereof. In addition, when the formulation is a soft capsule, it may comprise a vegetable oil, wax, fat, or semi-solid or liquid polyol. In addition, when the formulation is a solution or syrup, it may comprise water, polyol, glycerol, vegetable oil, or the like.

The pharmaceutical composition according to the present invention may further comprise a preservative, a stabilizer, a wetting agent, an emulsifier, a solubilizing agent, a sweetening agent, a colorant, an osmotic pressure regulator, an antioxidant, or the like, in addition to the carrier.

The pharmaceutical composition of the present invention may be administered in a pharmaceutically effective amount, and the term "pharmaceutically effective amount" refers to an amount sufficient for treating a disease at a reasonable benefit/risk ratio applicable to all medical treatments, and the effective dosage level may vary depending on a variety of factors comprising the type and severity of the disease of the patient, the activity of the drug, the sensitivity of the patient to the drug, the administration time, administration route and excretion rate of the composition according to the present invention, the treatment period, and drugs used concurrently therewith, along with other factors well-known in the pharmaceutical field. The pharmaceutical composition of the present invention may be administered as a single therapeutic agent or in combination with other therapeutic agents, may be administered sequentially or simultaneously with a conventional therapeutic agent, and may be administered in single or multiple doses. Taking into consideration these factors, it is important to administer the minimum amount sufficient to achieve maximum efficacy without side effects, and such an amount can be easily determined by those skilled in the art.

The pharmaceutical composition according to the present invention may be administered orally or parenterally. Parenteral administration may be intravenous injection, subcutaneous injection, intramuscular injection, intraperitoneal injection, endothelial administration, topical administration, intranasal administration, pulmonary administration, rectal administration or the like. Upon oral administration, since proteins or peptides are digested, an oral composition may be coated with an active drug or formulated so as to protect the same from degradation in the stomach. In addition, the composition may be administered using any device capable of delivering the active substance to target cells.

The method of administering the pharmaceutical composition according to the present invention may be easily selected depending on the formulation, and may be administered orally or parenterally. The dosage may vary depending on the patient's age, gender, weight, severity of the disease, and route of administration.

In the present invention, the coronavirus infection is most preferably SARS-CoV-2 infection (COVID-19).

In the present invention, the pharmaceutical composition may be used in combination with other compositions or methods for treating coronavirus infections.

In another aspect, the present invention is directed to the use of the receptor-binding domain variant and/or the fusion protein for the manufacture of a pharmaceutical composition for preventing or treating coronavirus infection.

In another aspect, the present invention is directed to a method for preventing or treating coronavirus infection, the method comprising administering the receptor-binding domain variant, the fusion protein, and/or the pharmaceutical composition to a subject.

In another aspect, the present invention is directed to the use of the receptor-binding domain variant and/or the fusion protein for the prevention or treatment of coronavirus infection.

In another aspect, the present invention is directed to a nucleic acid encoding the receptor-binding domain variant and/or the fusion protein.

The nucleic acid used herein may be present in a cell or a cell lysate, or may be present in a partially purified form or in a substantially pure form. The nucleic acid may be "isolated" or "become substantially pure" when purified from other cellular components or other contaminants, for example, from nucleic acids or proteins of other cells, by standard techniques comprising, for example, alkaline/SDS treatment, CsCl banding, column chromatography, agarose gel electrophoresis and other techniques well known in the art. The nucleic acid of the present invention may be, for example, DNA or RNA.

In another aspect, the present invention is directed to a recombinant vector comprising the nucleic acid according to the present invention.

Any vector known in the art can be appropriately selected and used as the recombinant vector by those skilled in the art without limitation, so long as it is capable of inducing the expression of a protein encoded by the receptor-binding domain variant or the fusion protein. For example, when *E. coli* is used as a host, vectors comprising T7 series (T7A1, T7A2, T7A3, etc.), lac, lacUV5, temperature-dependent (λphoA), phoB, rmB, tac, trc, trp or 1PL promoters may be used. When yeast is used as a host, vectors comprising the ADH1, AOX1, GAL1, GAL10, PGK or TDH3 promoter may be used, and when *Bacillus* is used as a host, vectors comprising the P2 promoter may be used. These are provided only as representative embodiments, and in addition to the vectors comprising the promoters, any vector can be appropriately selected from various vectors known in the art by those skilled in the art without limitation, so long as it is suitable for a host as a vector comprising a promoter for inducing the expression of the fusion protein according to the present invention.

As used herein, the term "vector" means a DNA product comprising a DNA sequence operably linked to a suitable regulatory sequence capable of expressing the DNA in a suitable host. Vectors may be plasmids, phage particles or simply potential genomic inserts. When transformed into a suitable host, vectors may be replicated or perform functions independent of the host genomes, or some thereof may be integrated with the genomes. A plasmid is currently the most commonly used form of vector, and thus the terms "plasmid" and "vector" are often used interchangeably. However, the present invention encompasses other forms of vectors that are known in the art or have the same functions as those known in the art. Protein expression vectors used in *E. coli* comprise: pET family vectors from Novagen, Inc (USA); pBAD family vectors from Invitrogen Corp. (USA); pHCE or pCOLD vectors from Takara Bio Inc. (Japan); and pACE family vectors from GenoFocus Inc. (South Korea). In *Bacillus subtilis,* a gene of interest can be inserted into a specific part of the genome to realize protein expression, or a pHT-family vector of MoBiTech (Germany) can be used. Even in fungi and yeast, protein expression is possible using genome insertion or self-replicating vectors. A plant protein expression vector using a T-DNA system such as *Agrobacterium tumefaciens* or *Agrobacterium rhizogenes* can be used. Typical expression vectors for expression in mammalian cell cultures are based on, for example, pRK5 (EP 307,247), pSV16B (WO 91/08291), and pVL1392 (Pharmingen).

As used herein, the term "expression control sequence" means a DNA sequence essential for the expression of a coding sequence operably linked to a particular host organism. Such a control sequence comprises promoters for conducting transcription, operator sequences for controlling such transcription, sequences for encoding suitable mRNA ribosome-binding sites, and sequences for controlling the termination of transcription and translation. For example, control sequences suitable for prokaryotes comprise promoters, optionally operator sequences, and ribosome-binding sites. Control sequences suitable for eukaryotic cells comprise promoters, polyadenylation signals, and enhancers. The factor that has the greatest impact on the expression level of a gene in a plasmid is the promoter. SRα promoters, *cytomegalovirus-derived* promoters and the like are preferably used as promoters for high expression.

Any of a wide variety of expression control sequences may be used for the vector in order to express the DNA sequences of the present invention. Useful expression control sequences comprise, for example, early and late promoters of SV40 or adenovirus, the lac system, the trp system, the TAC or TRC system, T3 and T7 promoters, the major operator and promoter regions of phage lambda, control regions of fd code proteins, promoters of 3-phosphoglycerate kinase or other glycol lyases, promoters of phosphatase, such as Pho5, promoters of yeast alpha-mating systems, and other sequences having configurations and induction activity known to control gene expression of prokaryotic or eukaryotic cells or viruses and various combinations thereof. The T7 RNA polymerase promoter Φ may be useful for expressing proteins in *E. coli.*

When a nucleic acid sequence is "operably linked" when it is placed in a functional relationship with another nucleic acid sequence. This may be gene(s) and control sequence(s) linked in such a way so as to enable gene expression when a suitable molecule (*e.g.*, a transcriptional activator protein) is linked to the control sequence(s). For example, DNA for a pre-sequence or secretory leader is operably linked to DNA for a polypeptide when expressed as a pre-protein involved in the secretion of the polypeptide; a promoter or enhancer is operably linked to a coding sequence when it affects the transcription of the sequence; a ribosome-binding site is operably linked to a coding sequence when it affects the transcription of the sequence; or a ribosome-binding site is operably linked to a coding sequence when positioned to facilitate translation. Generally, the term "operably linked" means that the linked DNA sequence is in contact therewith, and that a secretory leader is in contact therewith and is present in the reading frame. However, the enhancer need not be in contact therewith. The linkage of these sequences is carried out by ligation (linkage) at convenient restriction enzyme sites. When no such site exists, a synthetic oligonucleotide adapter or a linker according to a conventional method is used.

As used herein, the term "expression vector" commonly refers to a recombinant carrier into which a fragment of heterologous DNA is inserted, and generally means a fragment of double-stranded DNA. Herein, "heterologous DNA" means xenogenous DNA that is not naturally found in the host cell. Once an expression vector is present in a host cell, it can replicate independently of the host chromosomal DNA, and several copies of the vector and inserted (heterologous) DNA thereof can be produced.

As is well known in the art, in order to increase the expression level of a transfected gene in a recombinant cell, the gene should be operably linked to a transcriptional or translational expression control sequence that functions in the selected expression host. Preferably, the expression control sequence and the corresponding gene are comprised in a single expression vector comprising both a bacterial selection marker and a replication origin. When the expression host is a eukaryotic cell, the expression vector should further comprise a useful expression marker in the eukaryotic expression host.

In another aspect, the present invention is directed to a host cell into which the nucleic acid encoding the receptor-binding domain variant or the fusion protein and/or the recombinant vector are introduced.

As used herein, the term "host cell" refers to an expression cell introduced with a gene or a recombinant vector to produce a protein or the like. The host cell may be used without limitation, as long as it is a cell capable of expressing the receptor-binding domain variant and/or the fusion protein of the present invention, and the host cell is preferably a eukaryotic cell, more preferably yeast, an insect cell, or an animal cell, and most preferably an animal cell. For example, the host cell is a CHO cell line or a HEK cell line mainly used for expression of a fusion protein, but is not limited thereto.

Various expression host/vector combinations may be used to express the receptor-binding domain variant and/or the fusion protein of the present invention. Suitable expression vectors for eukaryotic hosts comprise, for example, but are not limited to, expression control sequences derived from SV40, cow papillomavirus, adenovirus, adeno-associated virus, cytomegalovirus and retrovirus. Expression vectors that can be used for bacterial hosts comprise bacterial plasmids obtained from *Escherichia coli (E. coli),* such as pBluescript, pGEX2T, pUC vectors, col E1, pCR1, pBR322, pMB9 and derivatives thereof, plasmids having a wide host range such as RP4, phage DNA that can be exemplified by a wide variety of phage lambda derivatives such as λ NM989, and other DNA phages such as M13 and filamentous single-stranded DNA phages. Expression vectors useful for yeast cells comprise 2µ plasmids and derivatives thereof. A vector that is useful for insect cells is pVL 941.

The recombinant vector may be introduced into the host cell through transfection or transformation. As used herein, the term "transfection" means introducing DNA into a host and making the DNA replicable using an extrachromosomal factor or chromosomal integration. As used herein, the term "transformation" means that an expression vector is accommodated by the host cell, regardless of whether or not any coding sequence is actually expressed.

It should be understood that not all vectors function identically in expressing the DNA sequences of the present invention. Likewise, not all hosts function identically for the same expression system. However, those skilled in the art will be able to make appropriate selections from among a variety of vectors, expression control sequences and hosts without excessive burden of experimentation and without departing from the scope of the present invention. For example, selection of a vector should be carried out in consideration of a host because the vector should be replicated therein. The number of replications of the vector, the ability to control the number of replications, and the expression of other proteins encoded by the corresponding vector, such as the expression of antibiotic markers, should also be considered. In selecting the expression control sequence, a number of factors should be considered. For example, the relative strength of the sequence, controllability, and compatibility with the DNA sequences of the present invention should be considered, particularly in relation to possible secondary structures. The single-cell host may be selected in consideration of factors such as the selected vector, the toxicity of the product encoded by the DNA sequence of the present invention, secretion characteristics, the ability to accurately fold proteins, culture and fermentation factors, and ease of purification of the product encoded by the DNA sequence according to the present invention. Within the scope of these factors, those skilled in the art can select various vector/expression control sequences/host combinations capable of expressing the DNA sequences of the present invention in fermentation or large animal cultures. As a screening method for cloning cDNA of proteins through expression cloning, a binding method, a panning method, a film emulsion method or the like can be applied.

The gene and recombinant vector may be introduced into host cells through various methods known in the art. The gene encoding nucleic acid encoding the receptor-binding domain variant and/or the fusion protein of the present invention may be directly introduced into the genome of a host cell and present as a factor on a chromosome. It will be apparent to those skilled in the art to which the present invention pertains that even if the gene is inserted into the genomic chromosome of the host cell, it will have the same effect as when the recombinant vector is introduced into the host cell.

In another aspect, the present invention is directed to a method for producing a receptor-binding domain variant and/or a fusion protein comprising the same, the method comprising culturing the host cell.

When a recombinant expression vector capable of expressing the receptor-binding domain variant and/or the fusion protein comprising the same is introduced into a mammalian host cell, the receptor-binding domain variant and/or the fusion protein comprising the same can be produced by culturing the host cell for a period of time sufficient to allow expression of the fusion protein in the host cell, more preferably, culturing the host cells for a period of time sufficient to allow the receptor-binding domain variant and/or the fusion protein comprising the same to be secreted into the culture medium.

In some cases, the expressed the receptor-binding domain variant and/or the fusion protein comprising the same may be separated from the host cell and purified to homogeneity. The separation or purification of the receptor-binding domain variant and/or the fusion protein comprising the same can be carried out using separation and purification methods used for conventional proteins, for example, chromatography. The chromatography may, for example, comprise a combination of one or more selected from affinity chromatography, ion exchange chromatography, and hydrophobic chromatography, but is not limited thereto. A combination of chromatography with filtration, ultrafiltration, salting out, dialysis or the like may be used.

Although specific amino acid sequences and nucleotide sequences are described in the present invention, it will be apparent to those skilled in the art that amino acid sequences substantially identical to the enzymes to be implemented in the present invention and the nucleotide sequences encoding the same fall within the scope of the present invention. The term "substantially identical" comprises the case where the amino acid or nucleotide sequence is highly homologous and the case of a protein that shares structural characteristics regardless of the homology of the sequence or has the same function as that used in the present invention. The present invention may comprise an enzyme from which a portion of a sequence other than the sequence constituting the core of the present invention has been deleted or a fragment of a nucleotide sequence encoding the same, and may comprise all amino-acid or nucleotide sequences having the same function as that used in the present invention regardless of the length of the fragment.

### Example

Hereinafter, the present invention will be described in more detail with reference to the following examples. However, these examples are provided only for illustration of the present invention, and should not be construed as limiting the scope of the present invention.

### Example 1: Preparation of fusion protein comprising wild-type receptor-binding domain or variant

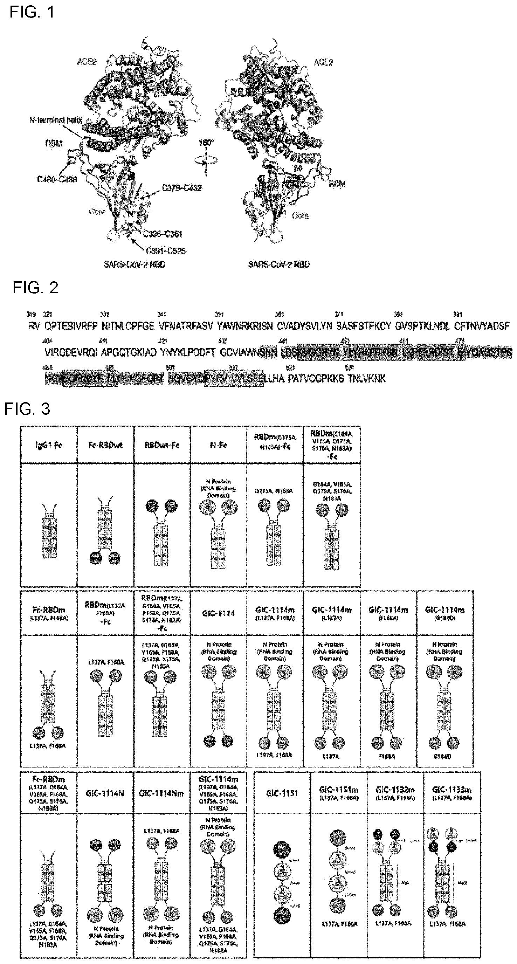

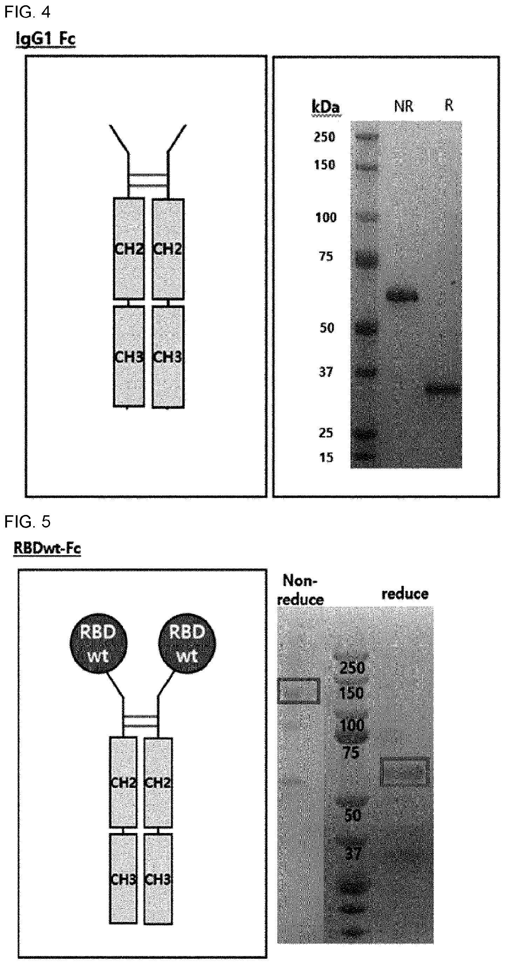

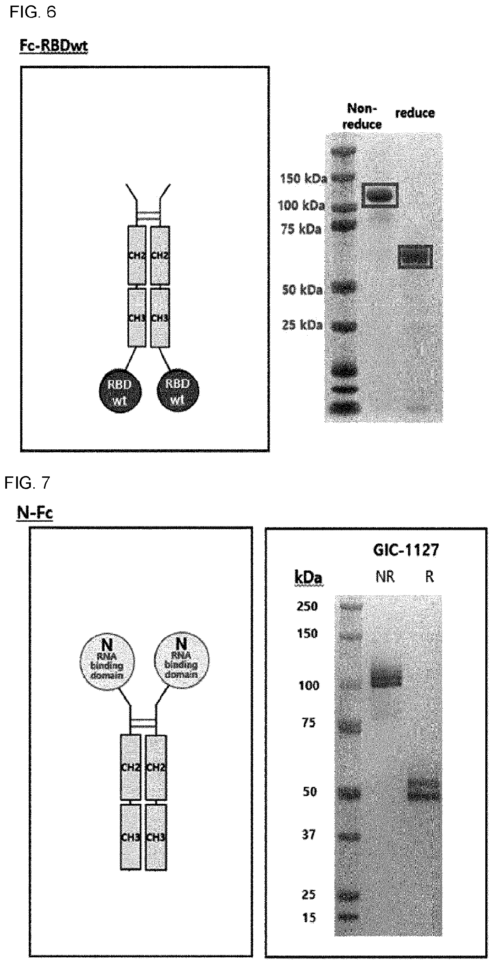

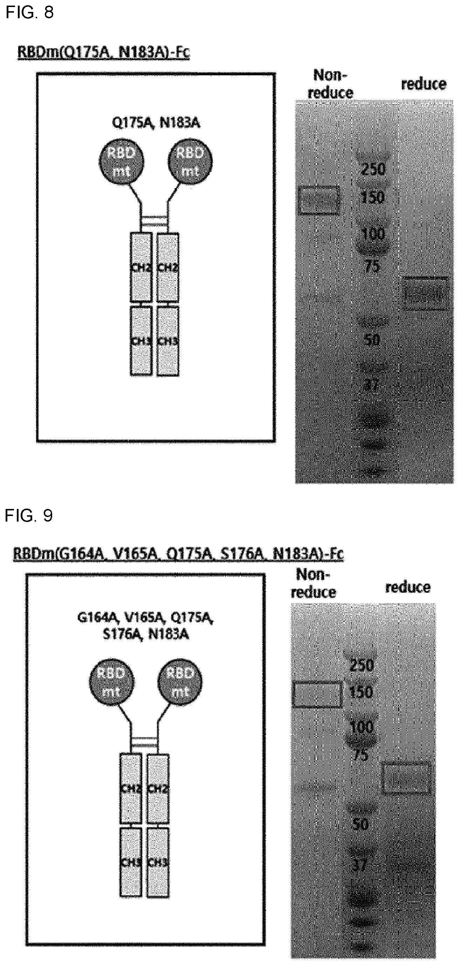

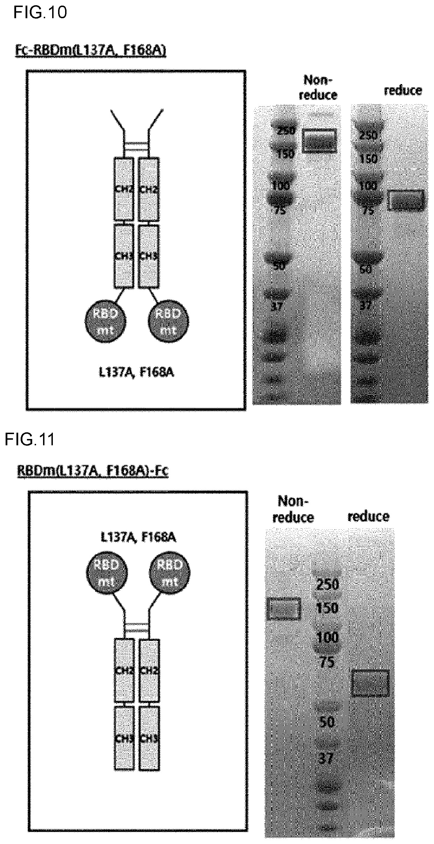

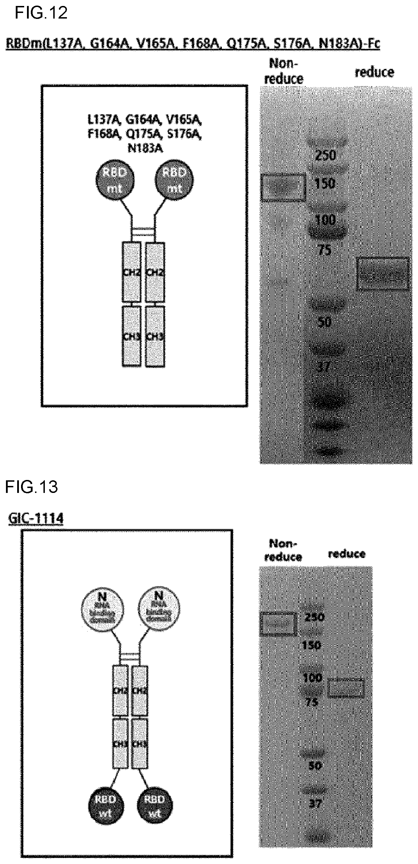

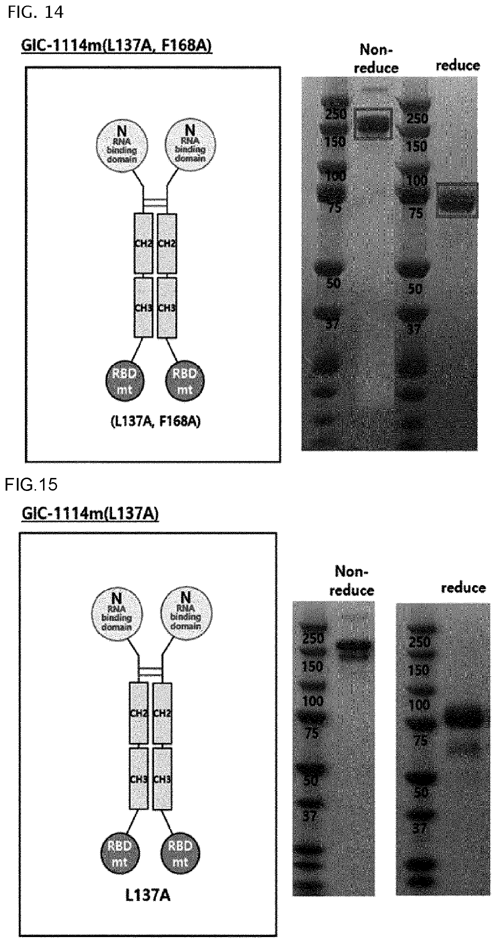

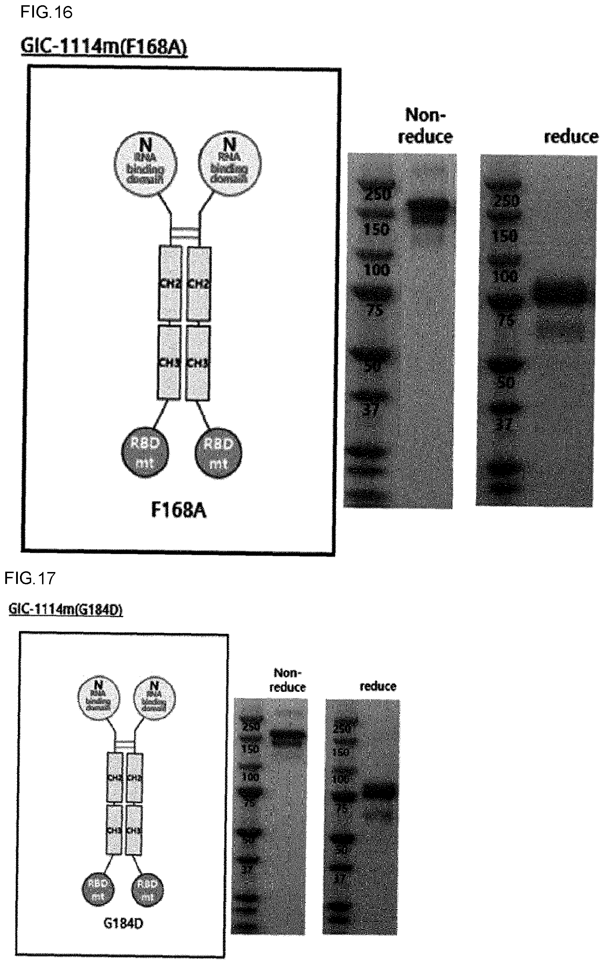

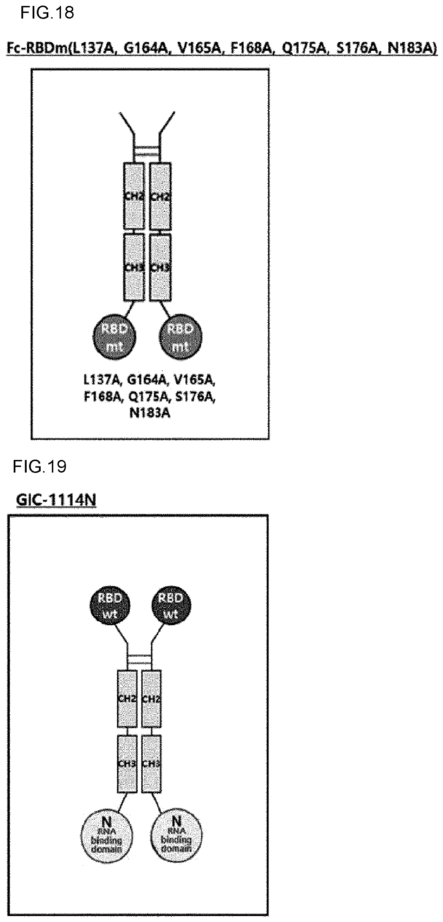

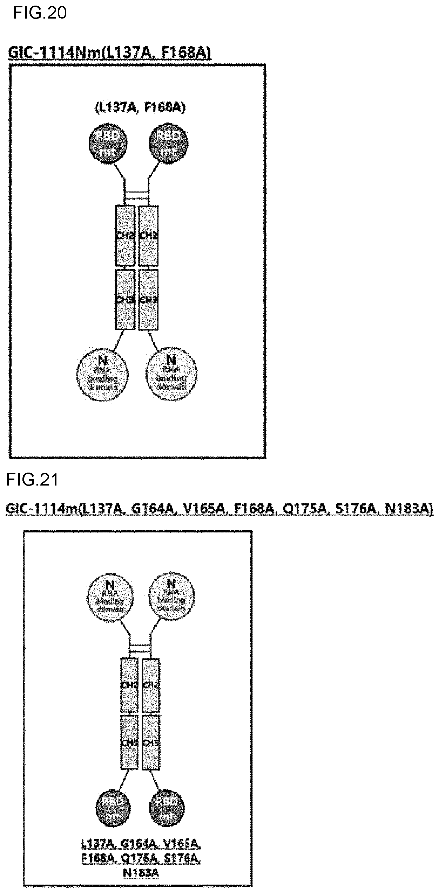

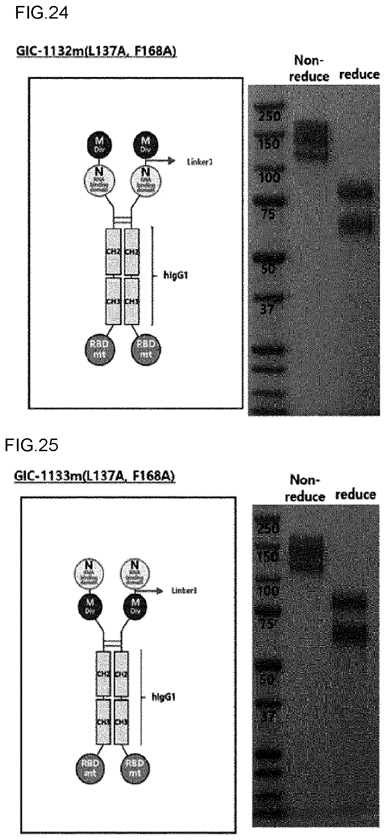

A control group and an experimental group were set as shown in FIG. 3, and a fusion protein (FIGS. 4 to 25) was produced in order to evaluate the ACE2-binding affinity and immunogenicity of the receptor-binding domain (RBD) variant. In order to produce a fusion protein, a wild-type RBD, a variant RBD, an IgG1 Fc domain, and a nucleocapsid protein (N protein) RNA-binding domain shown in Table 1 below were linked to design a fusion protein, and a nucleotide comprising a base sequence encoding the fusion protein was synthesized using the gBlock^{™} gene fragments service from Integrated DNA Technologies, and was loaded into pcDNA3.4 vector.

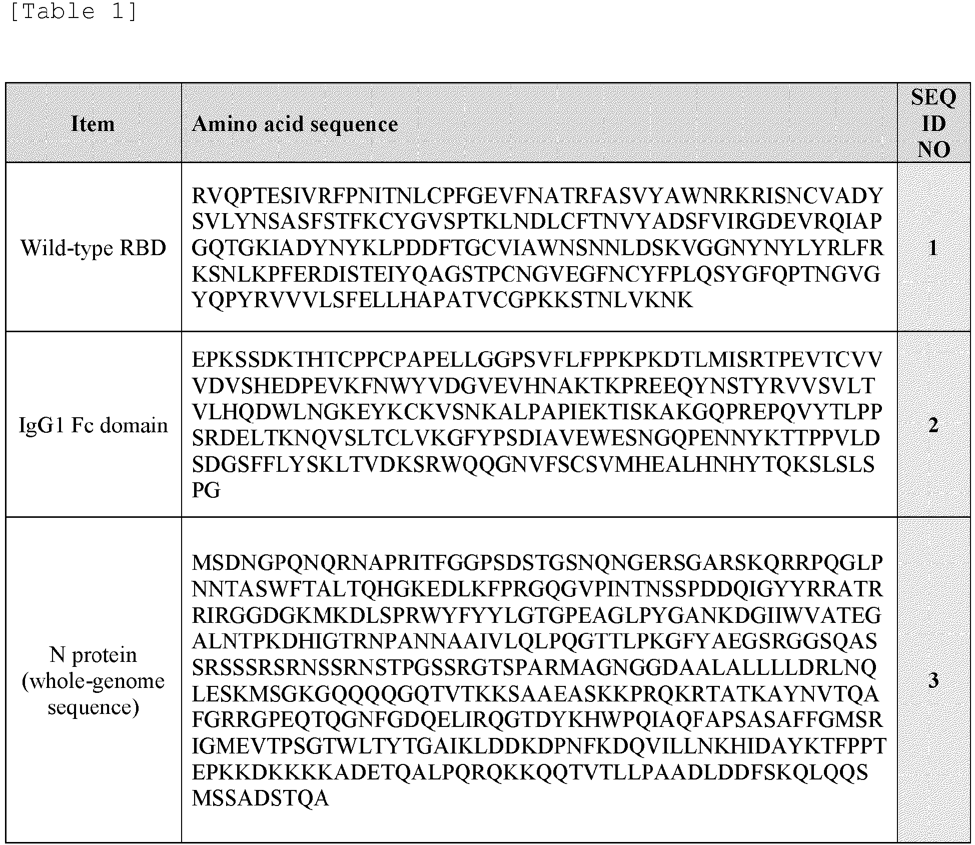

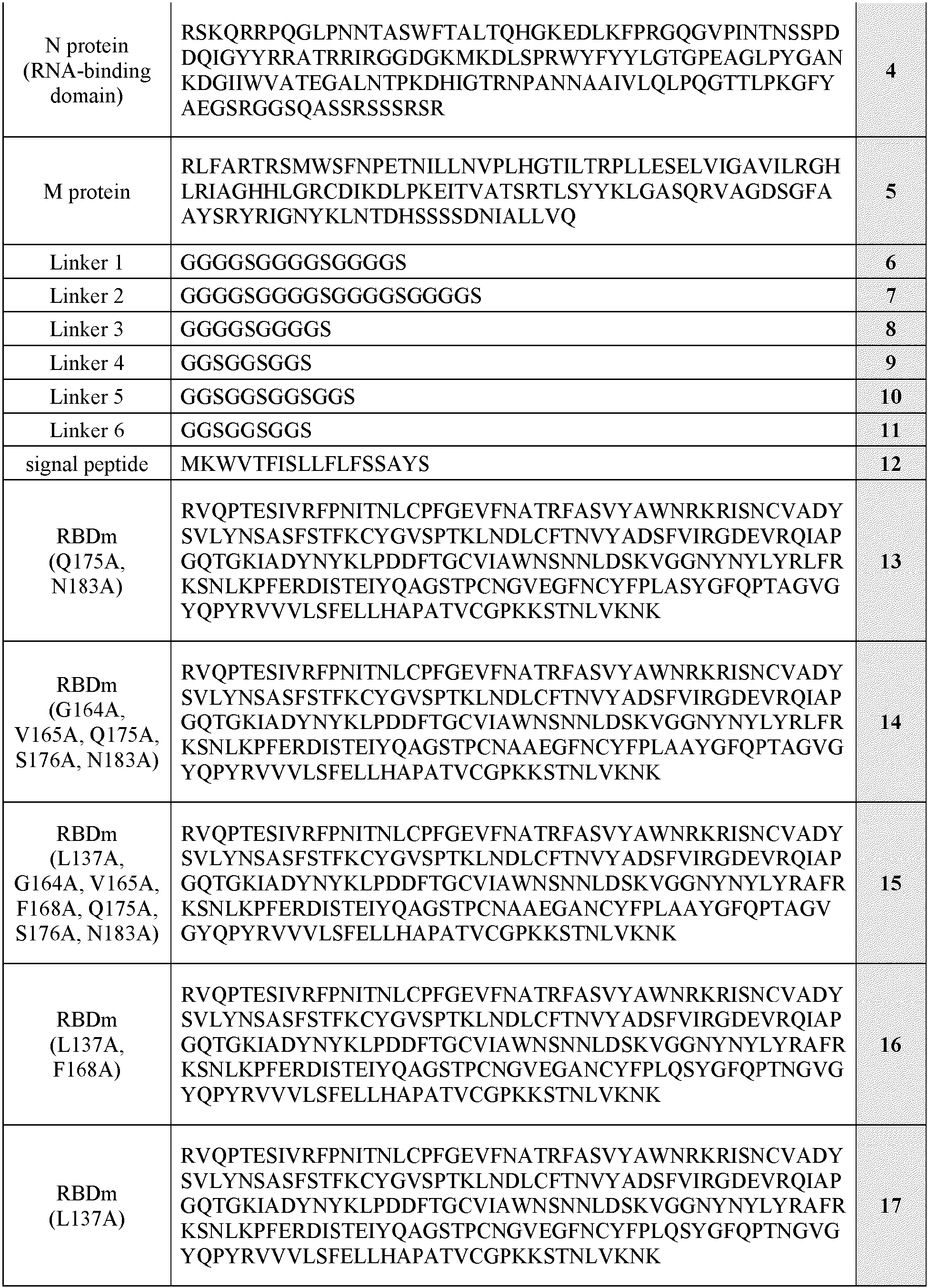

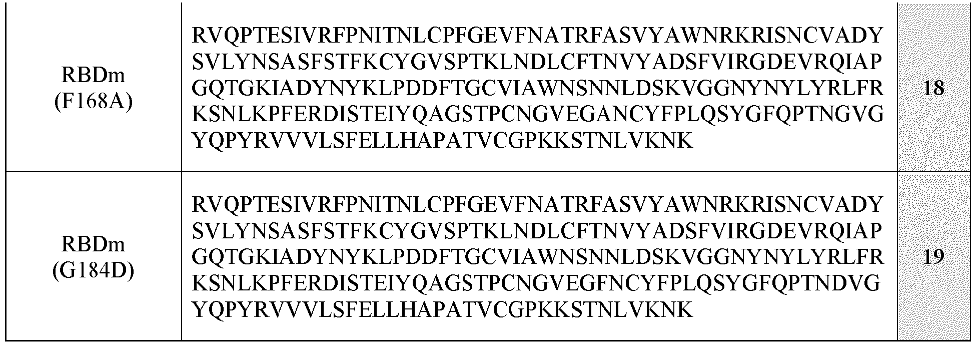

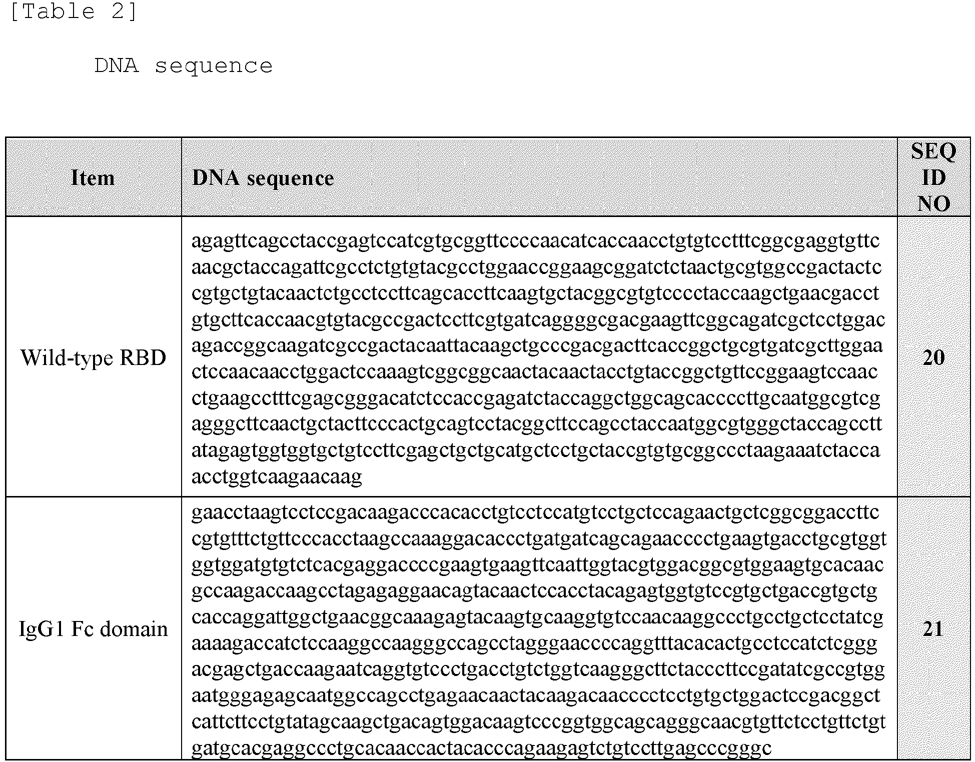

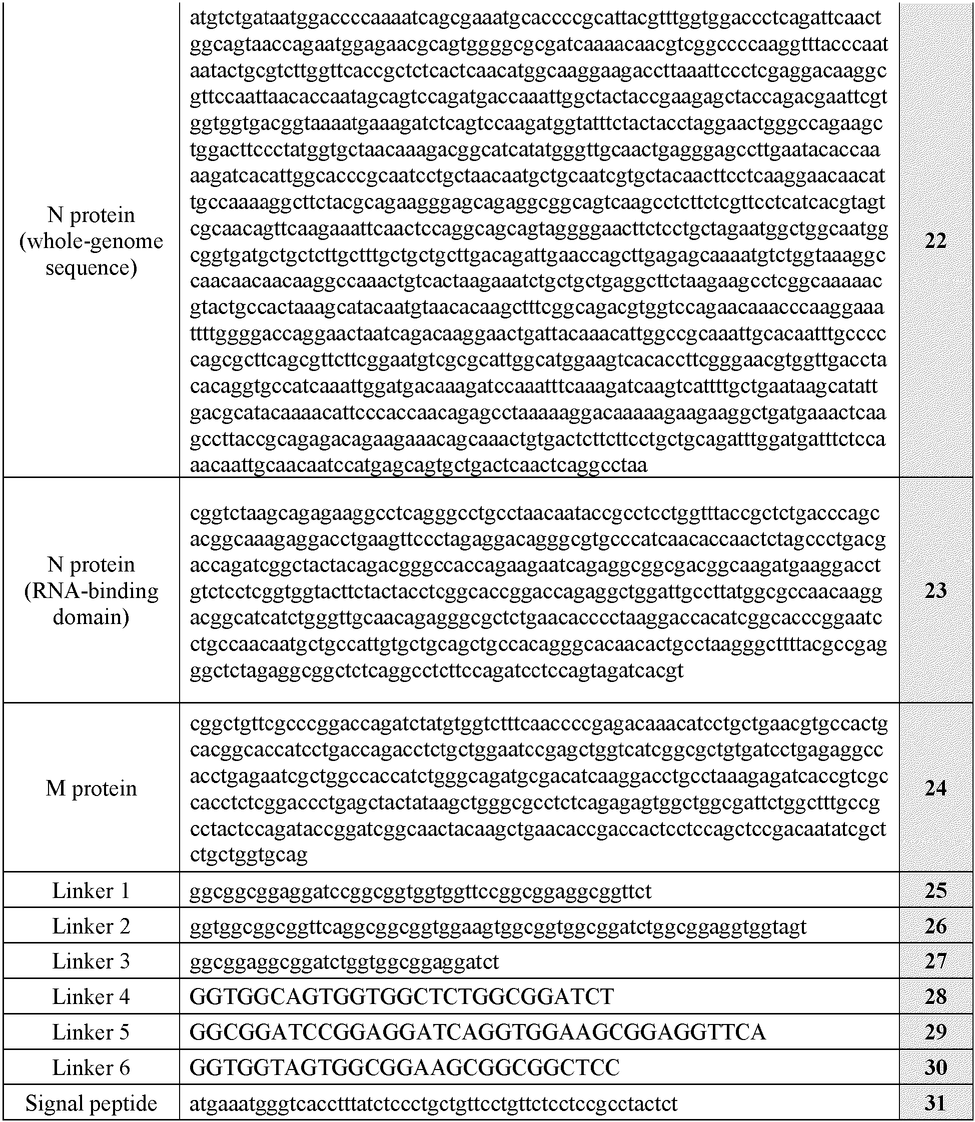

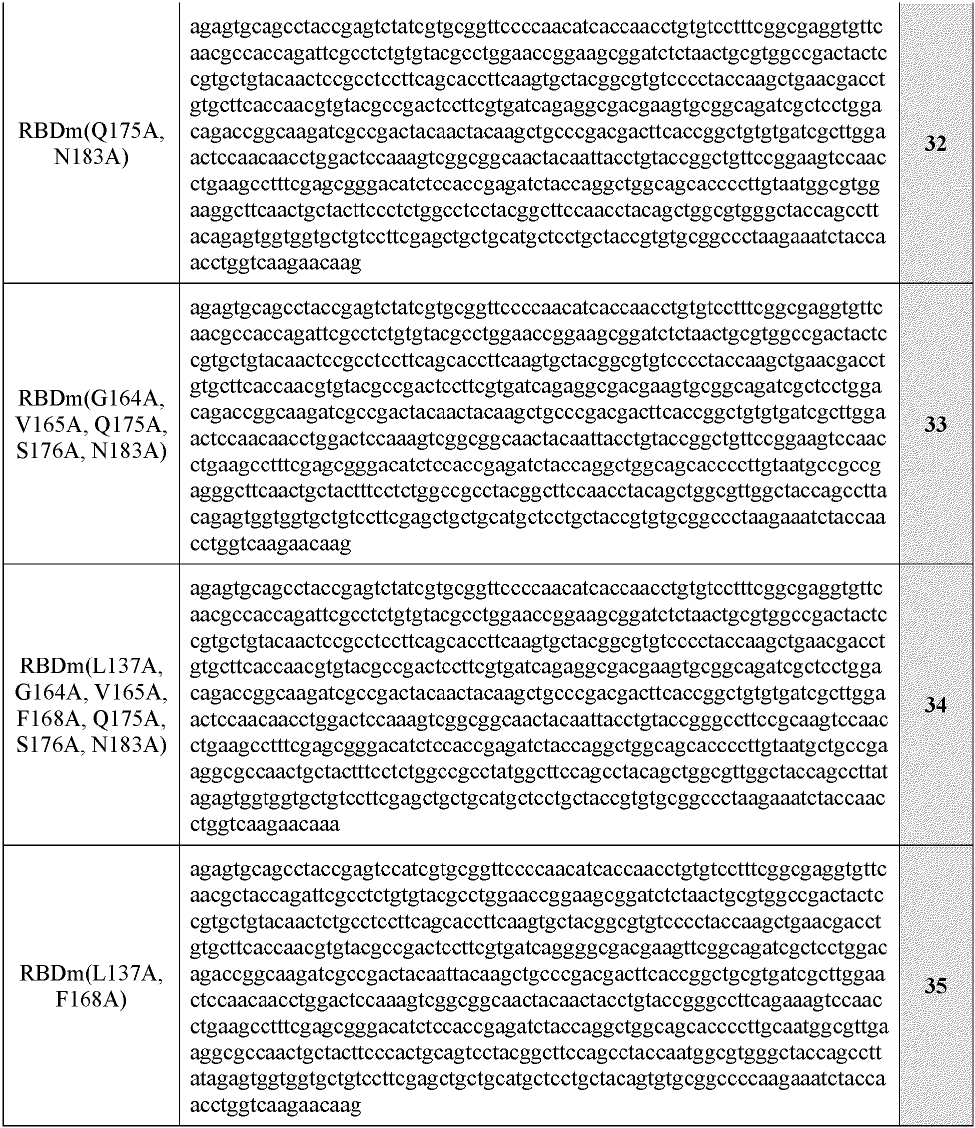

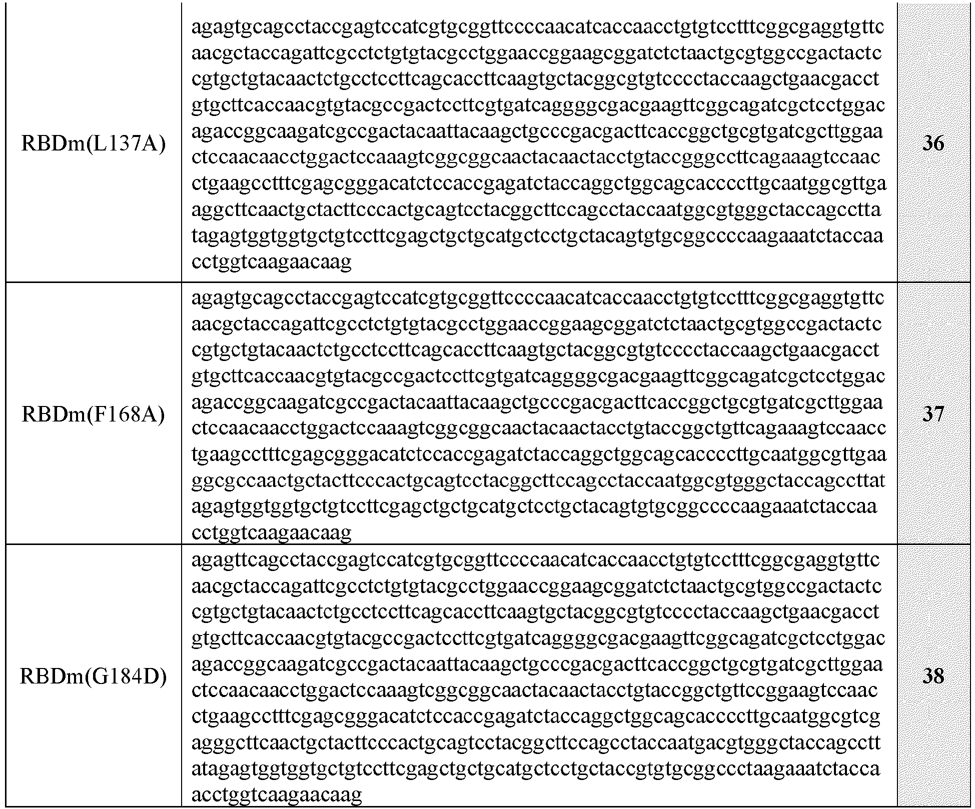

The produced vector was introduced into CHO cells (Expi-CHO^{™}) to express each fusion protein. After introduction of the vector, culture was performed under a 5% CO₂ environment at 37°C and at 125 RPM for 7 days, the culture medium was collected, and the fusion protein was purified.

Each fusion protein was purified by performing chromatography using a Mabselect Xtra resin. Equilibration was performed using a protein A binding buffer produced by Thermo Fisher Scientific Inc. Then, the supernatant filtered through a 0.22 µm filter was loaded onto the column, and the column was washed using a protein A binding buffer in a volume corresponding to 10 times the volume of the resin. Then, elution was performed using an IgG elution buffer produced by Thermo Fisher Scientific Inc. The fusion protein was collected in a collection tube comprising 20% 1M Tris-HCl at a pH of 9. Then, the buffer for collected fusion protein was replaced by PBS through dialysis.

Then, size exclusion chromatography was performed using a TSKgel G3000SWXL column (TOSOH Bioscience), and absorbance was measured at a wavelength of 214 nm to obtain a high-concentration fusion protein. At this time, formation of the separated and purified fusion protein was identified by performing SDS-PAGE under reducing (R) or non-reducing (NR) conditions, followed by staining with Coomassie blue.

GIC-1151 and GIC-1151m were purified through chromatography using a cation exchange resin (Capto S, GE). Equilibration was performed using a 50 mM Tris-HCl (pH 8.0) buffer. Then, the supernatant filtered through a 0.22 um filter was loaded onto the column, and the column was washed using a 50 mM Tris-HCl (pH 8.0) buffer in a volume corresponding to 10 times the volume of the resin. Then, elution was performed in divided sections by 5 mL using 50 mM Tris-HCl (pH 8.0) buffer and 1M NaCl (gradient) buffer. SDS-PAGE and staining with Coomassie blue were sequentially performed, and the fusion protein of the section identified as the expected size was collected, size exclusion chromatography was performed using a size exclusion resin (superdex 75pg 16/600, GE), and absorbance was measured at a wavelength of 214 nm to obtain a highly concentrated fusion protein. At this time, the separated and purified fusion protein was identified by performing SDS-PAGE under reducing (R) or non-reducing (NR) conditions and staining with Coomassie blue.

The amino acid sequence of the produced fusion protein and the nucleic acid sequence encoding the same are shown in Tables 3 and 4 below.

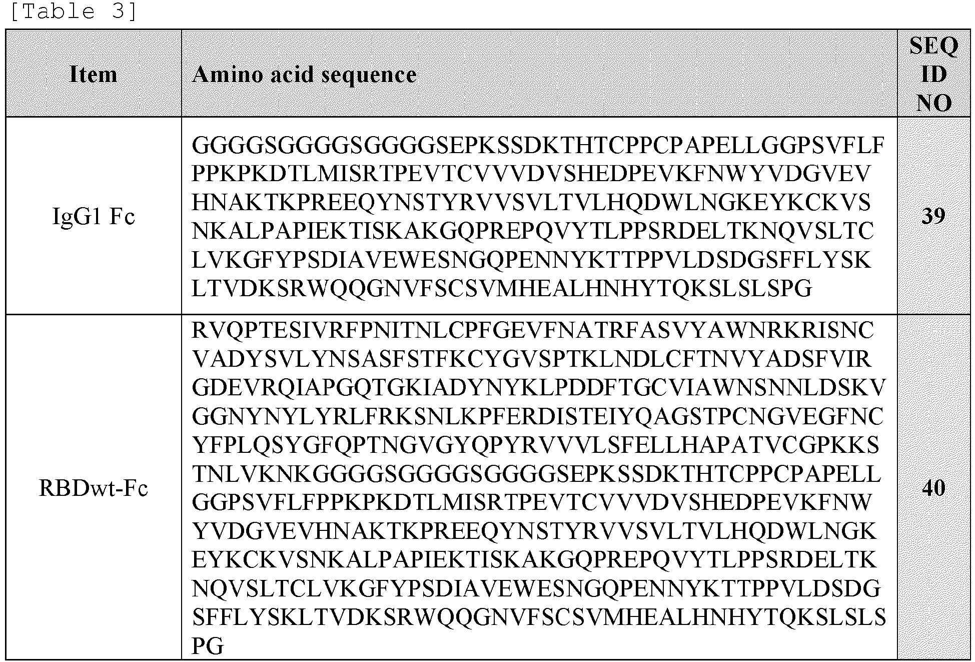

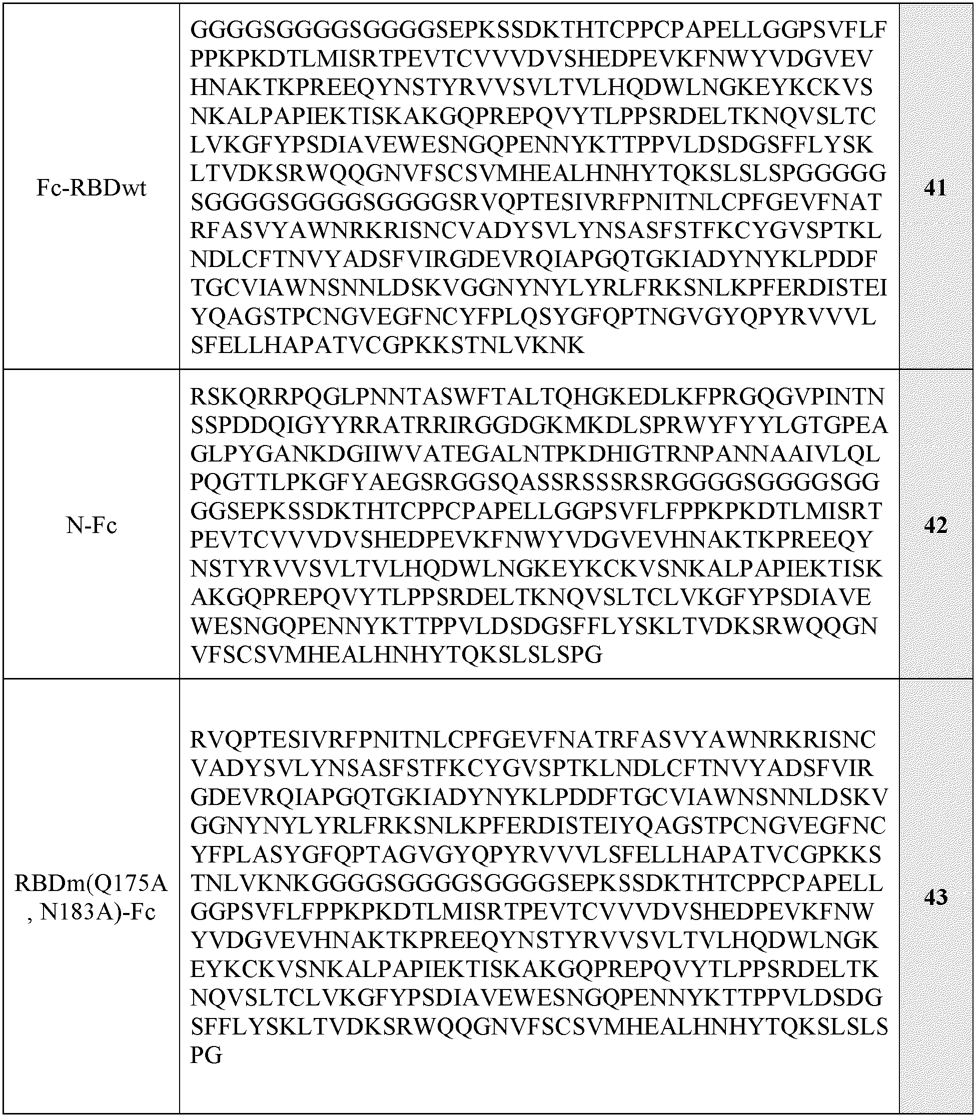

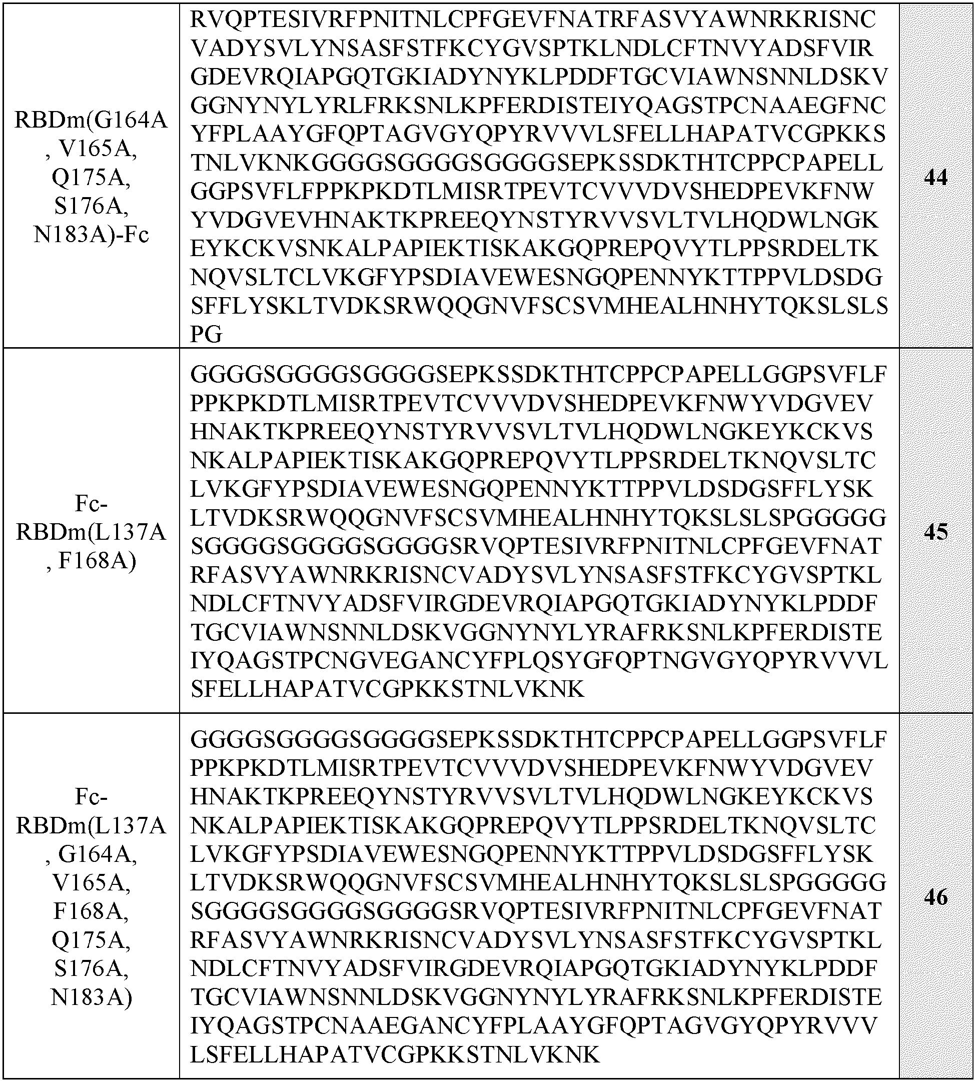

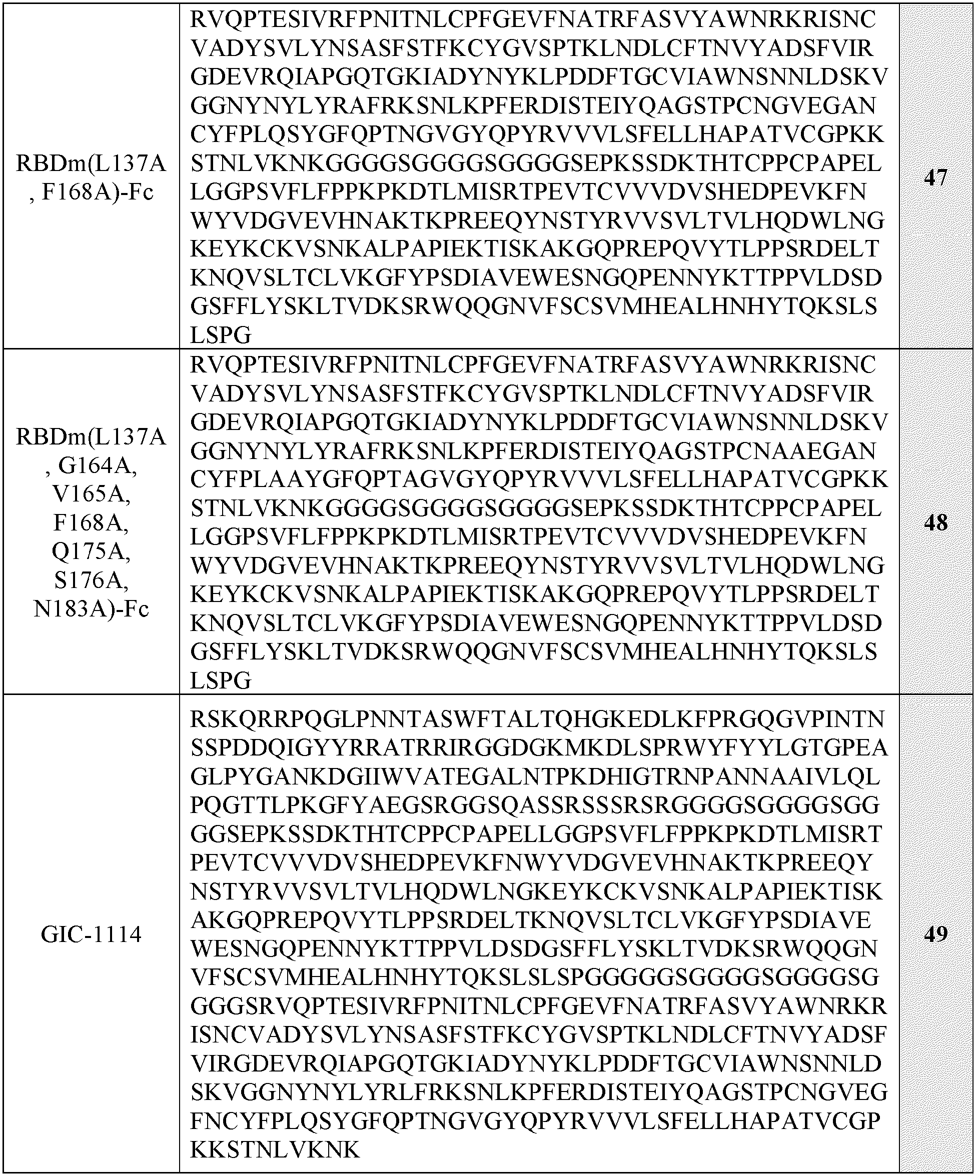

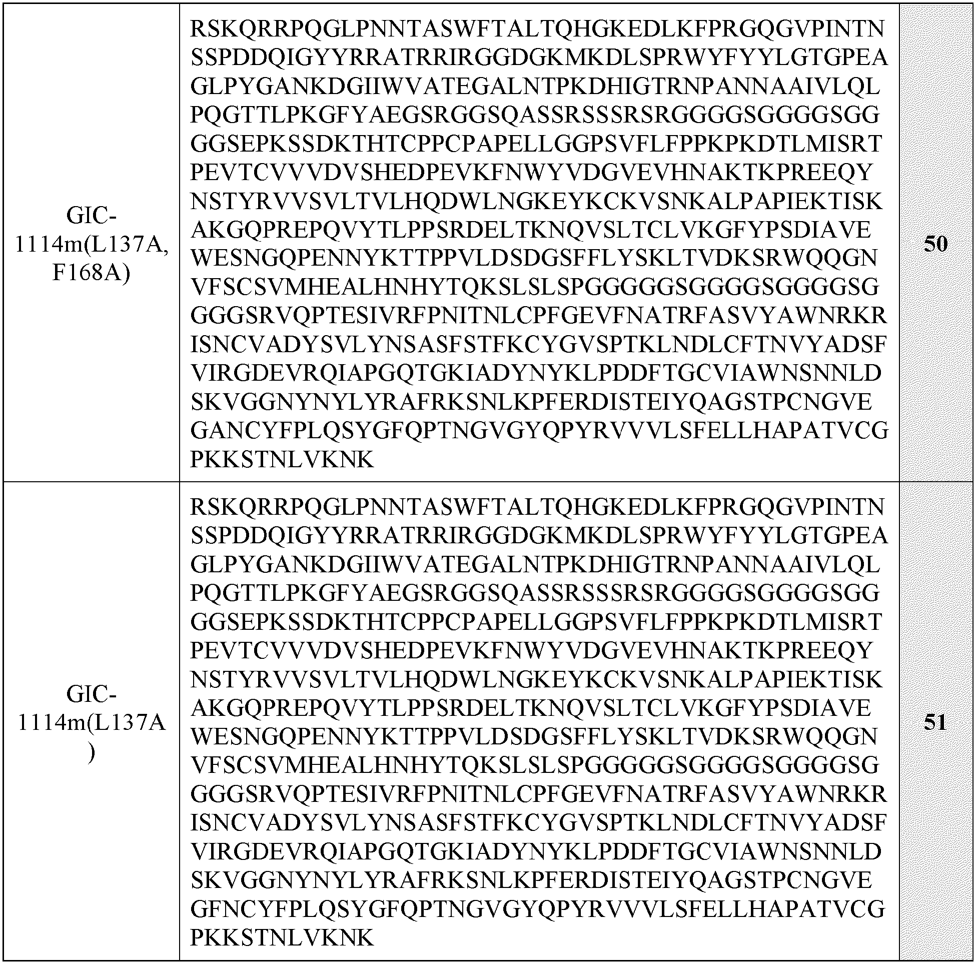

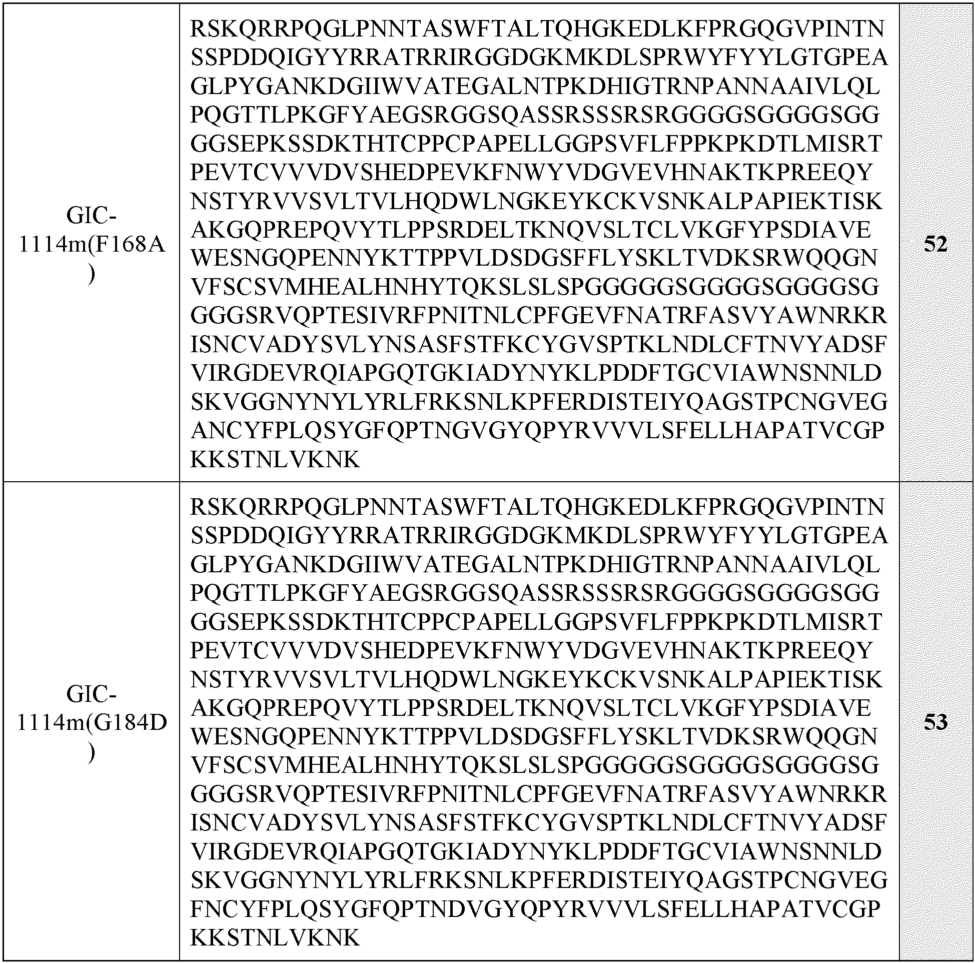

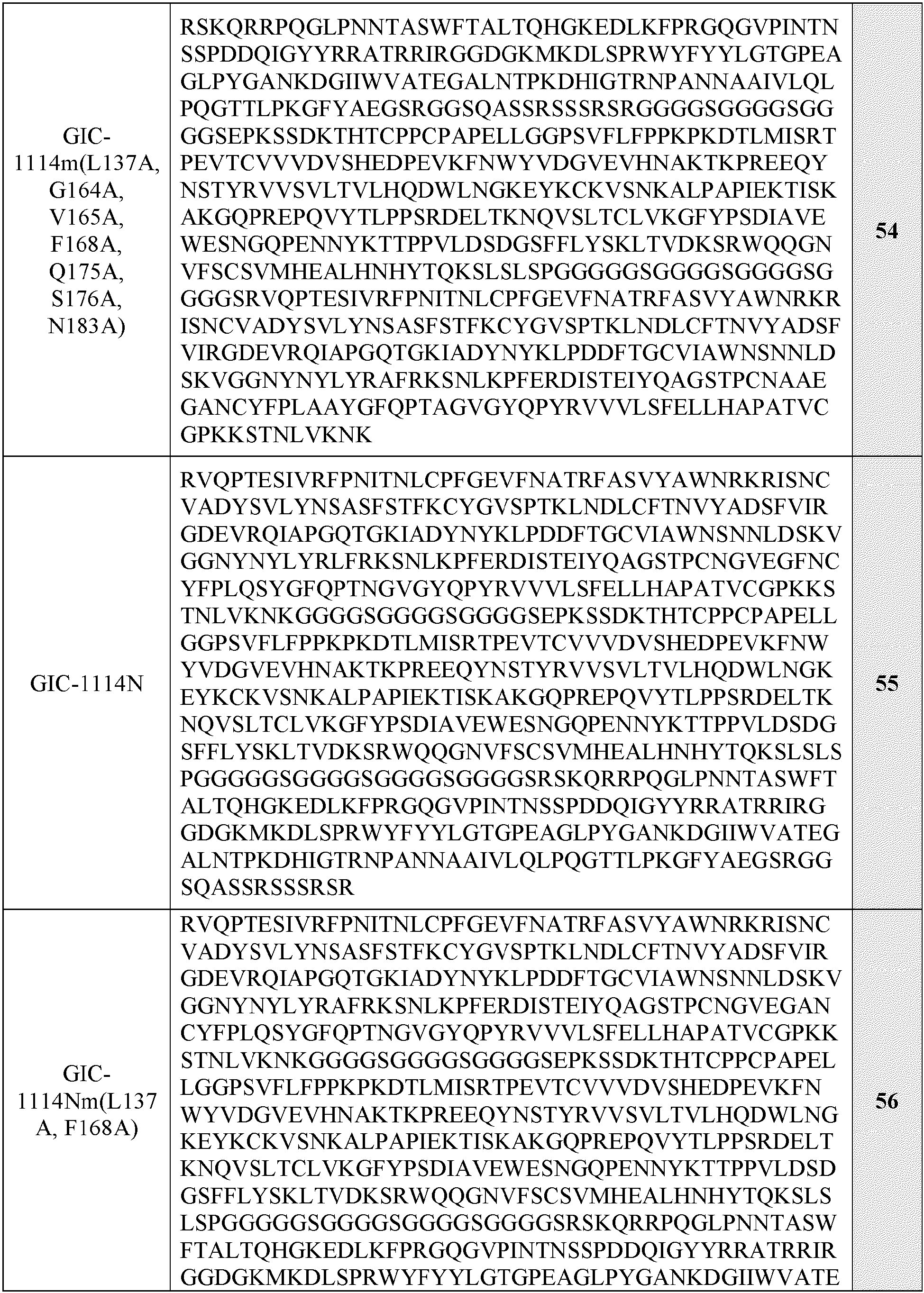

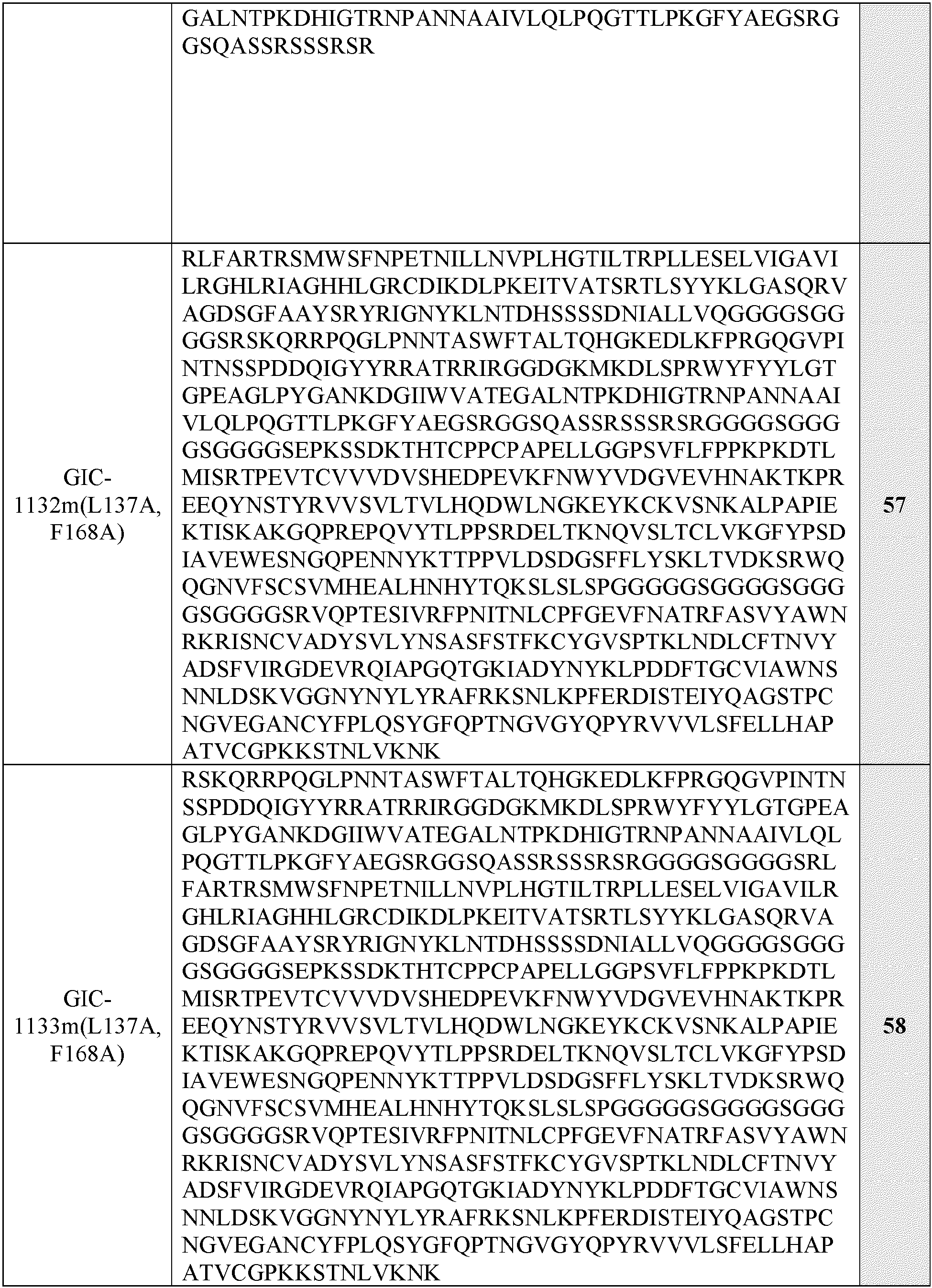

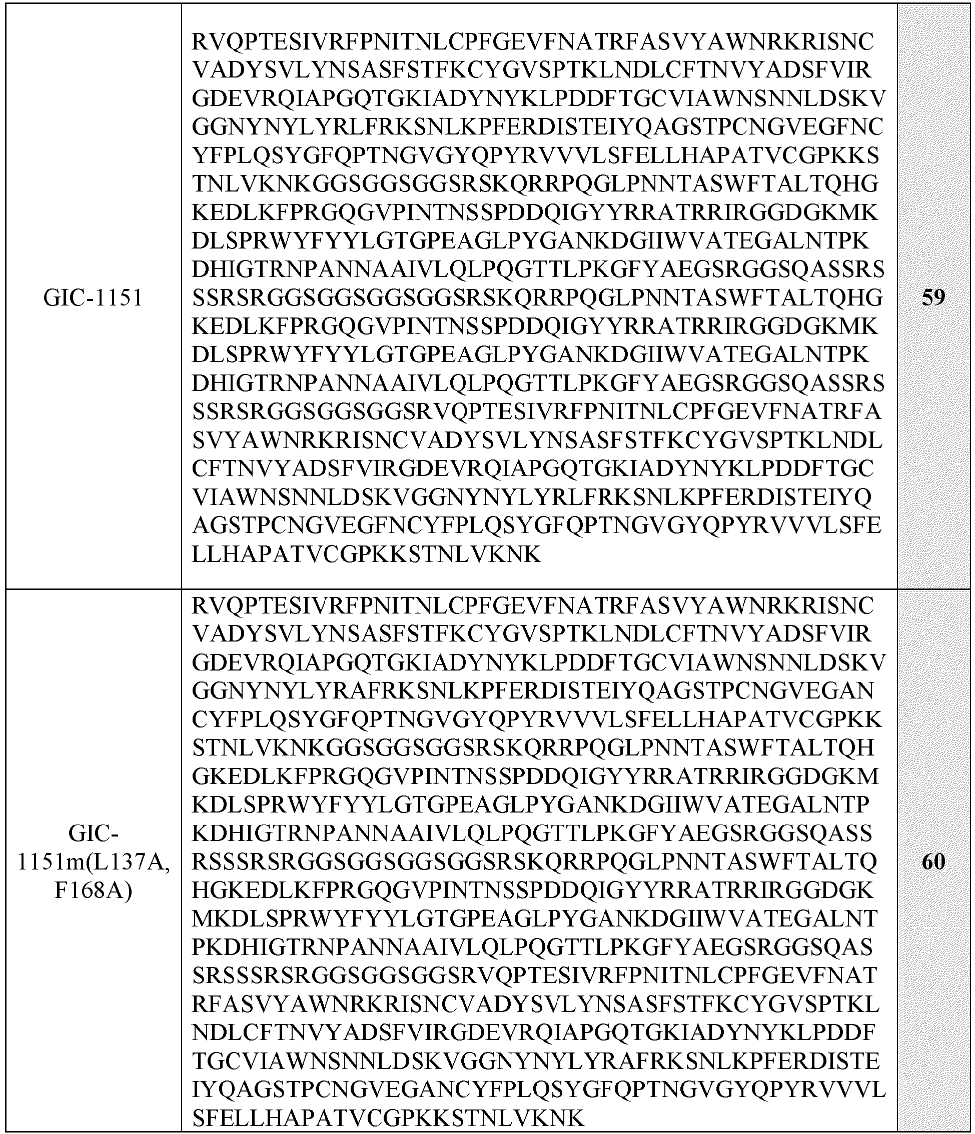

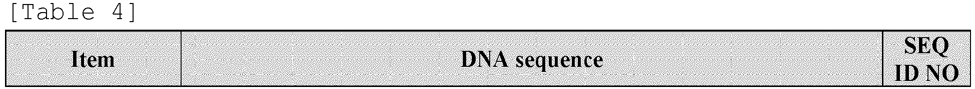

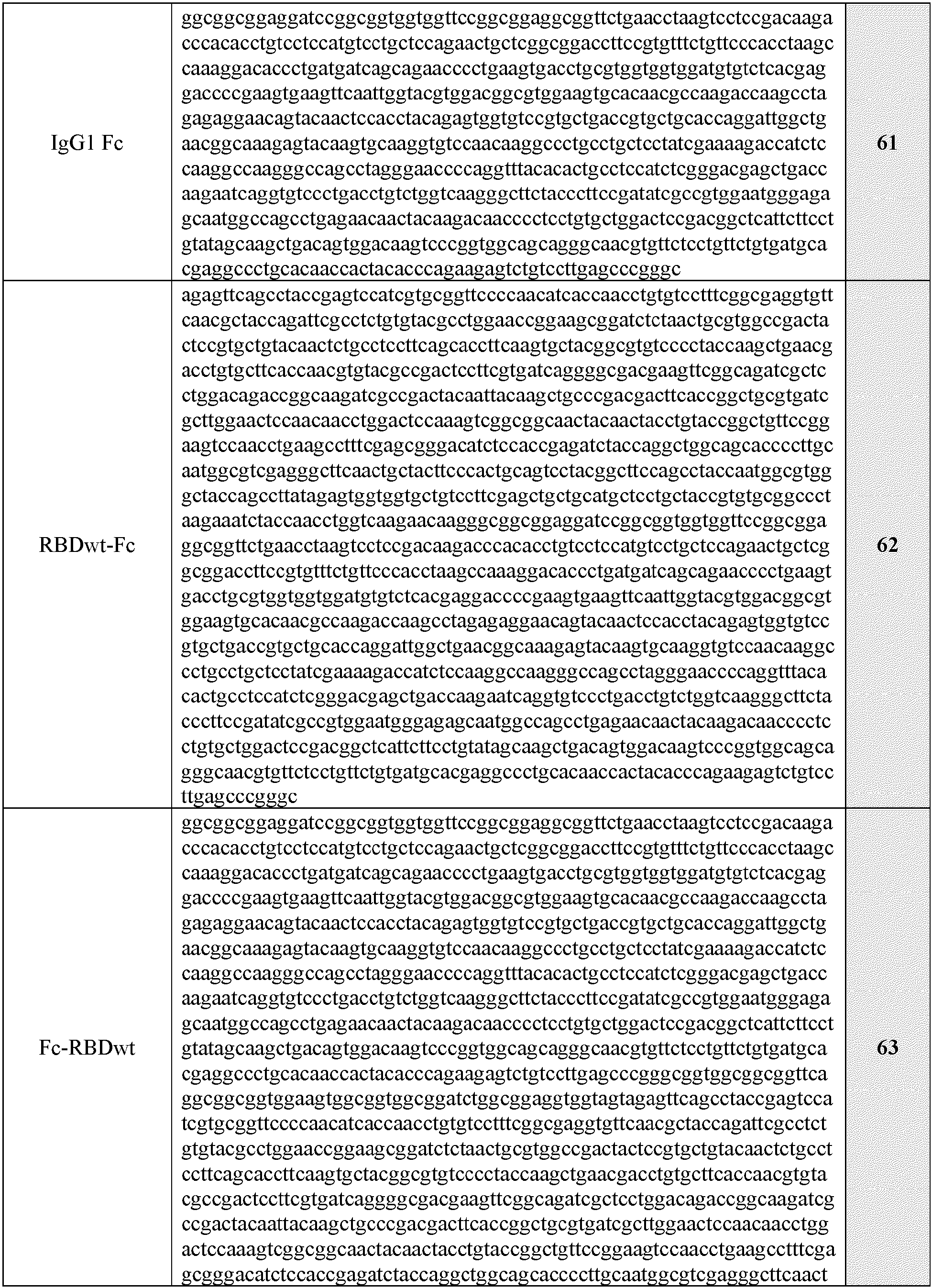

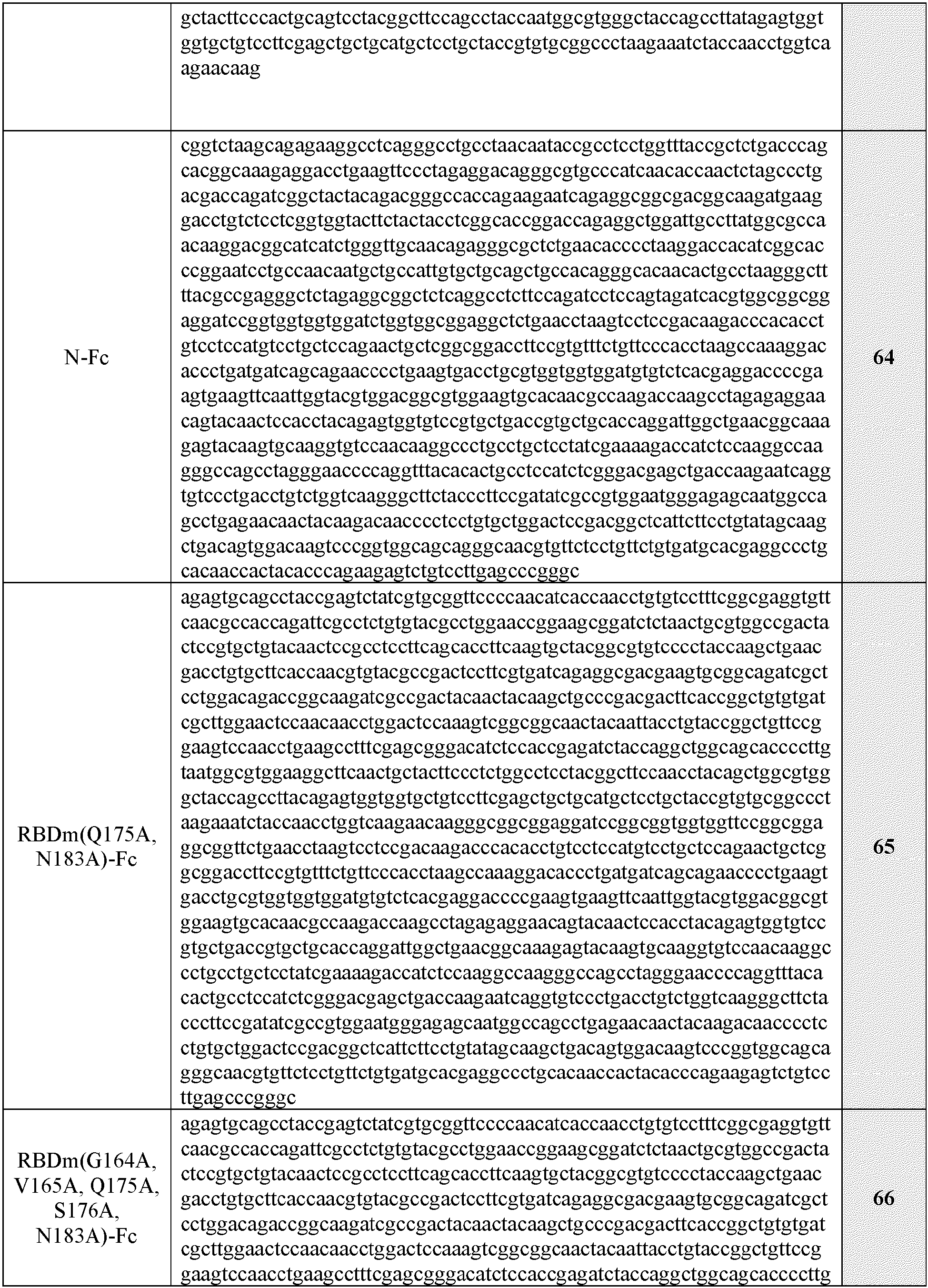

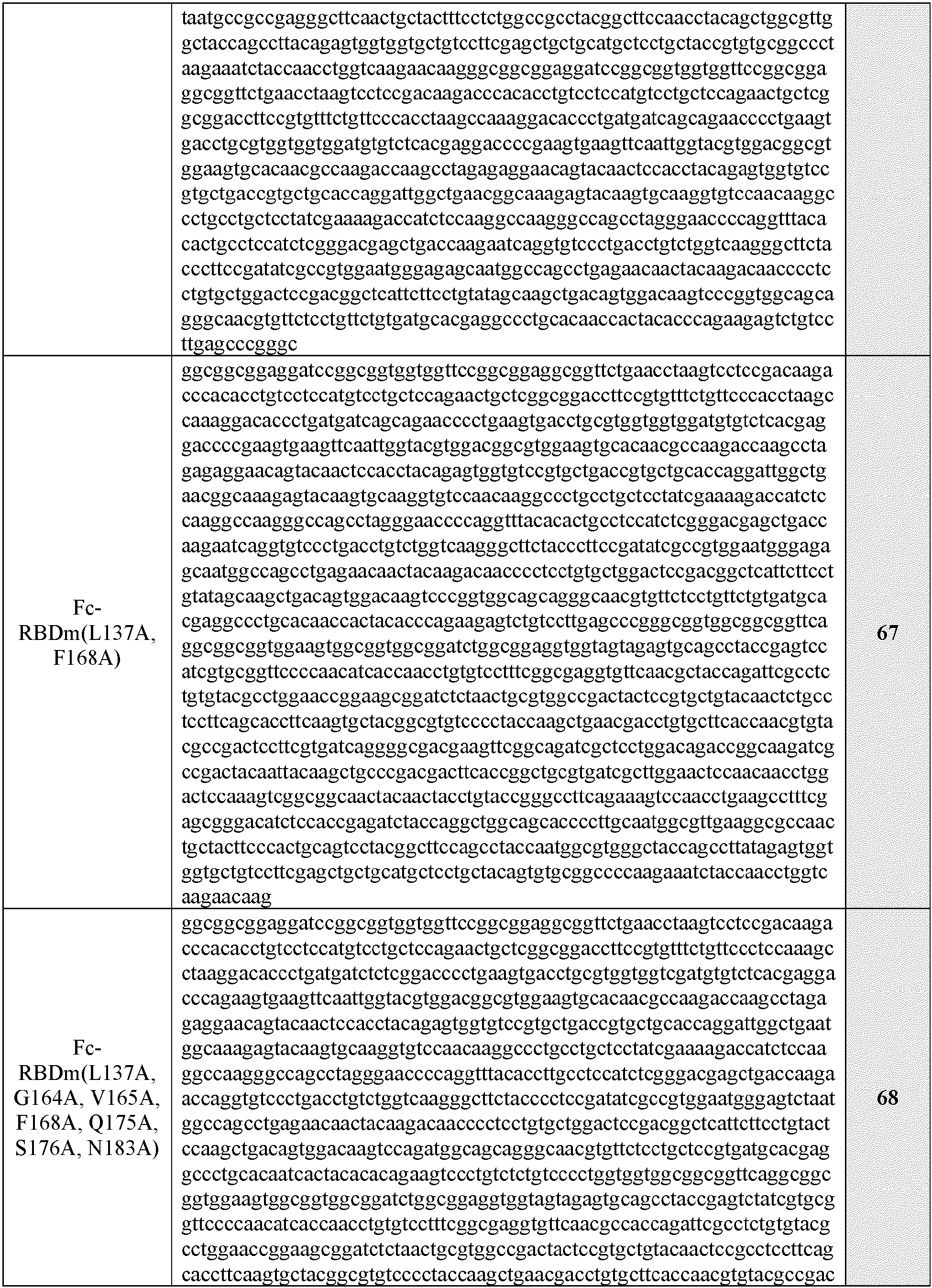

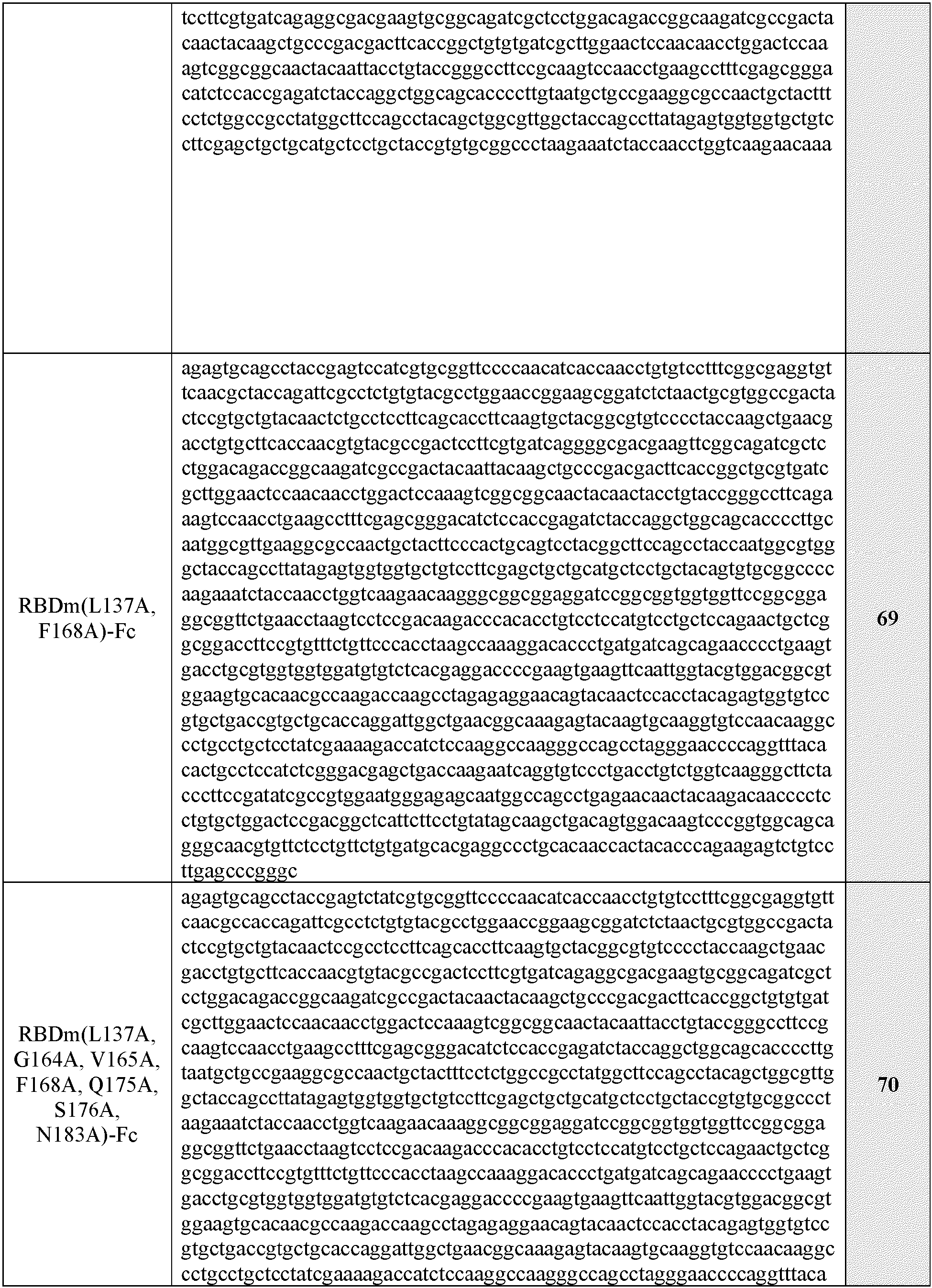

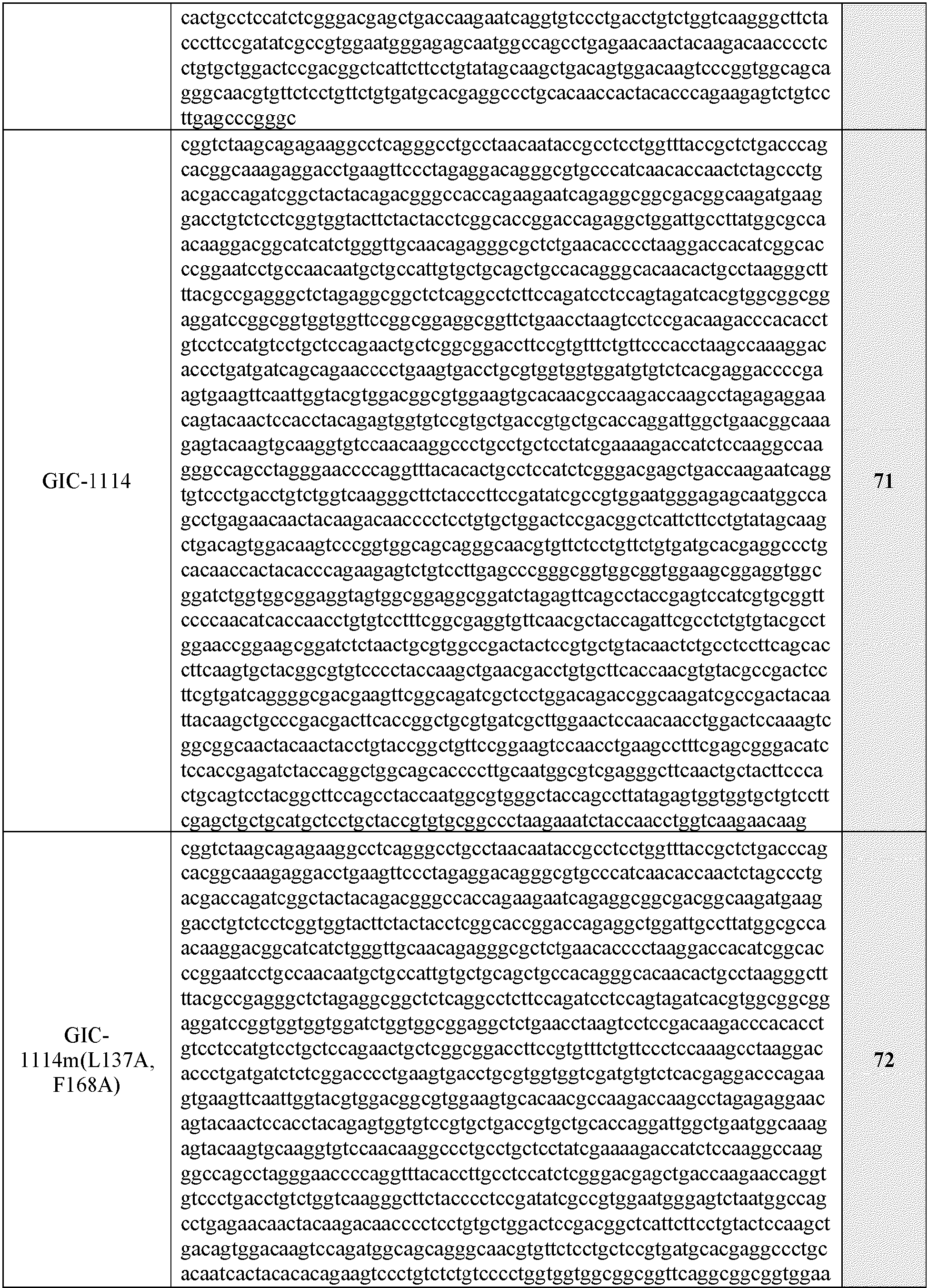

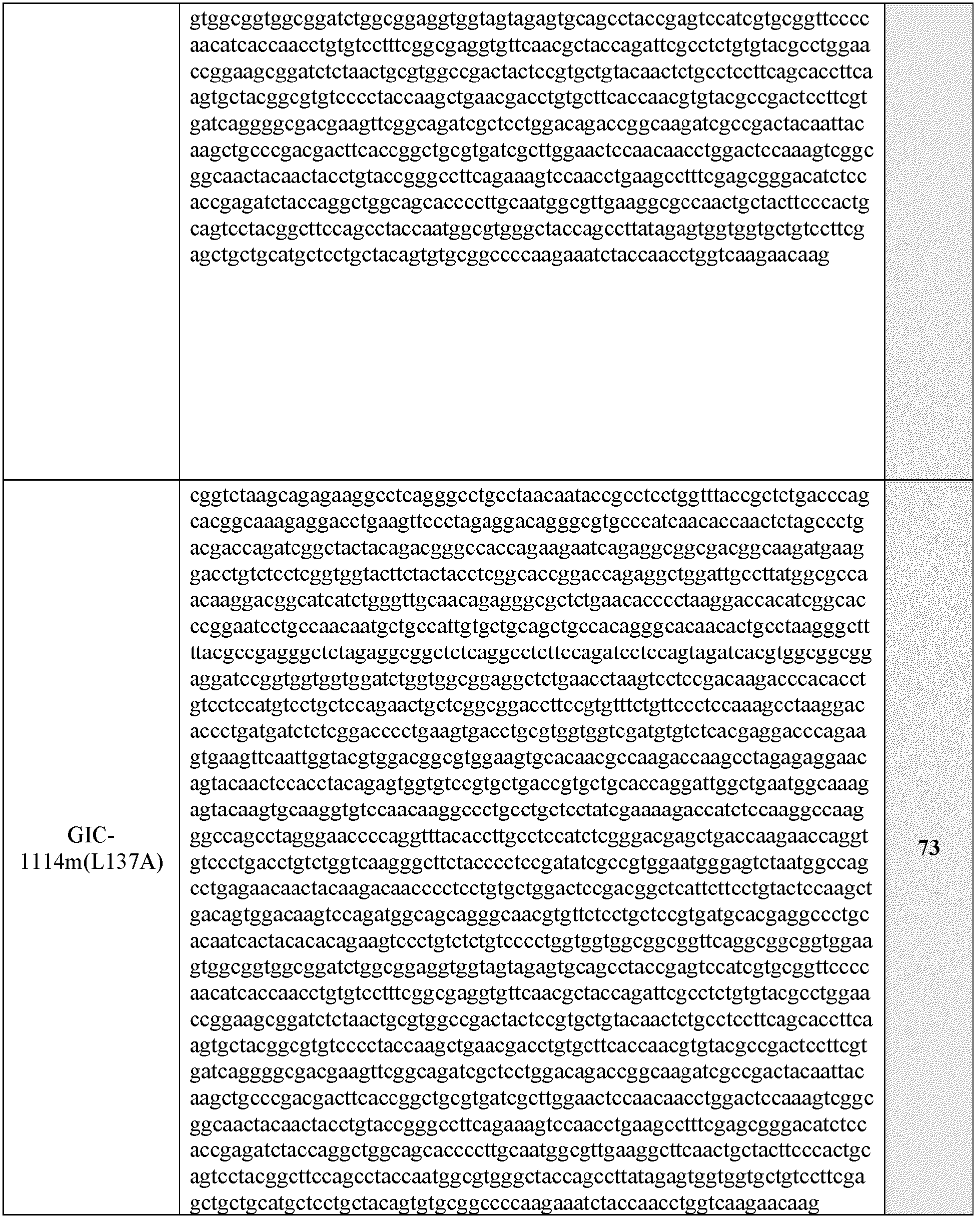

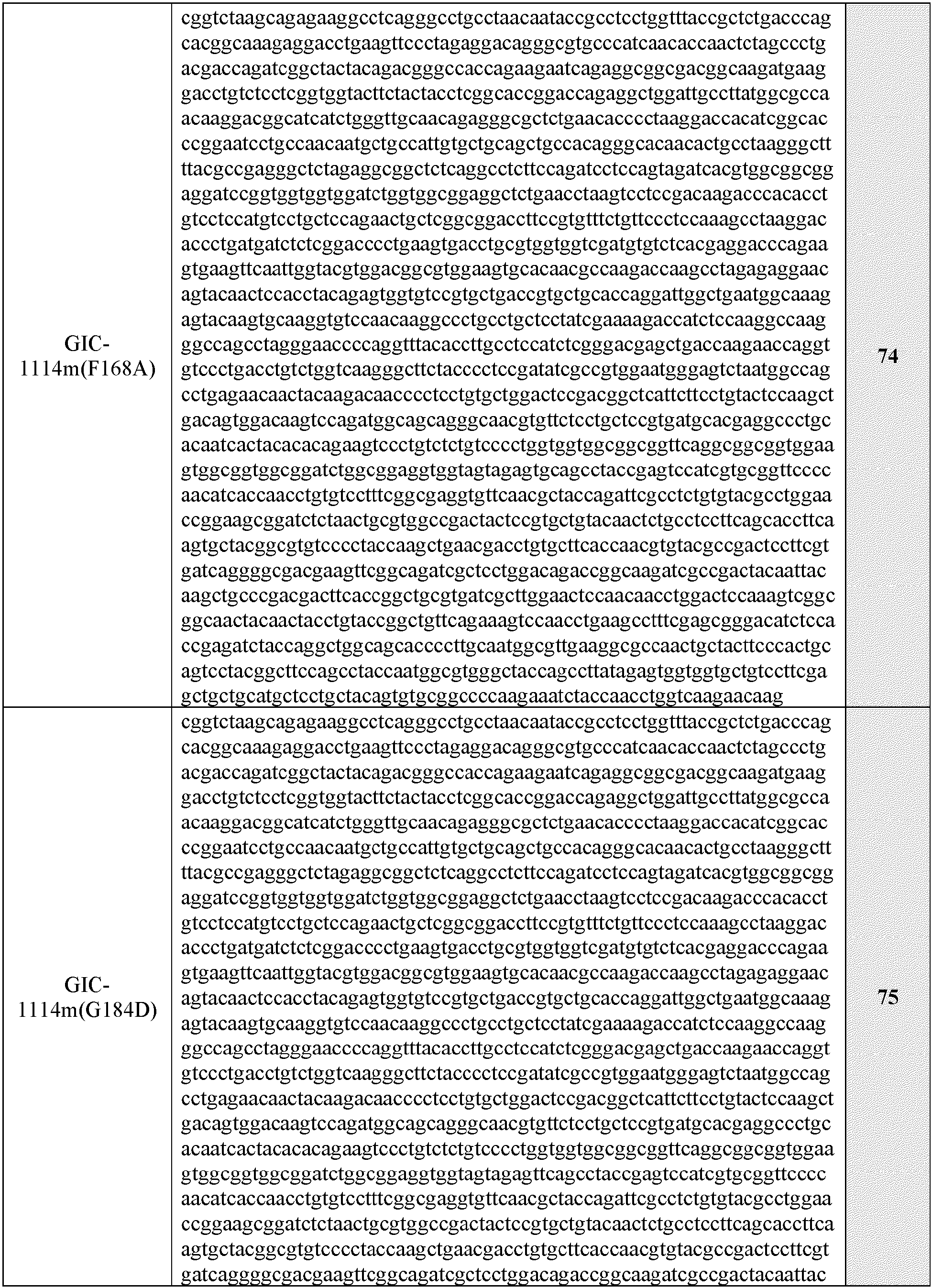

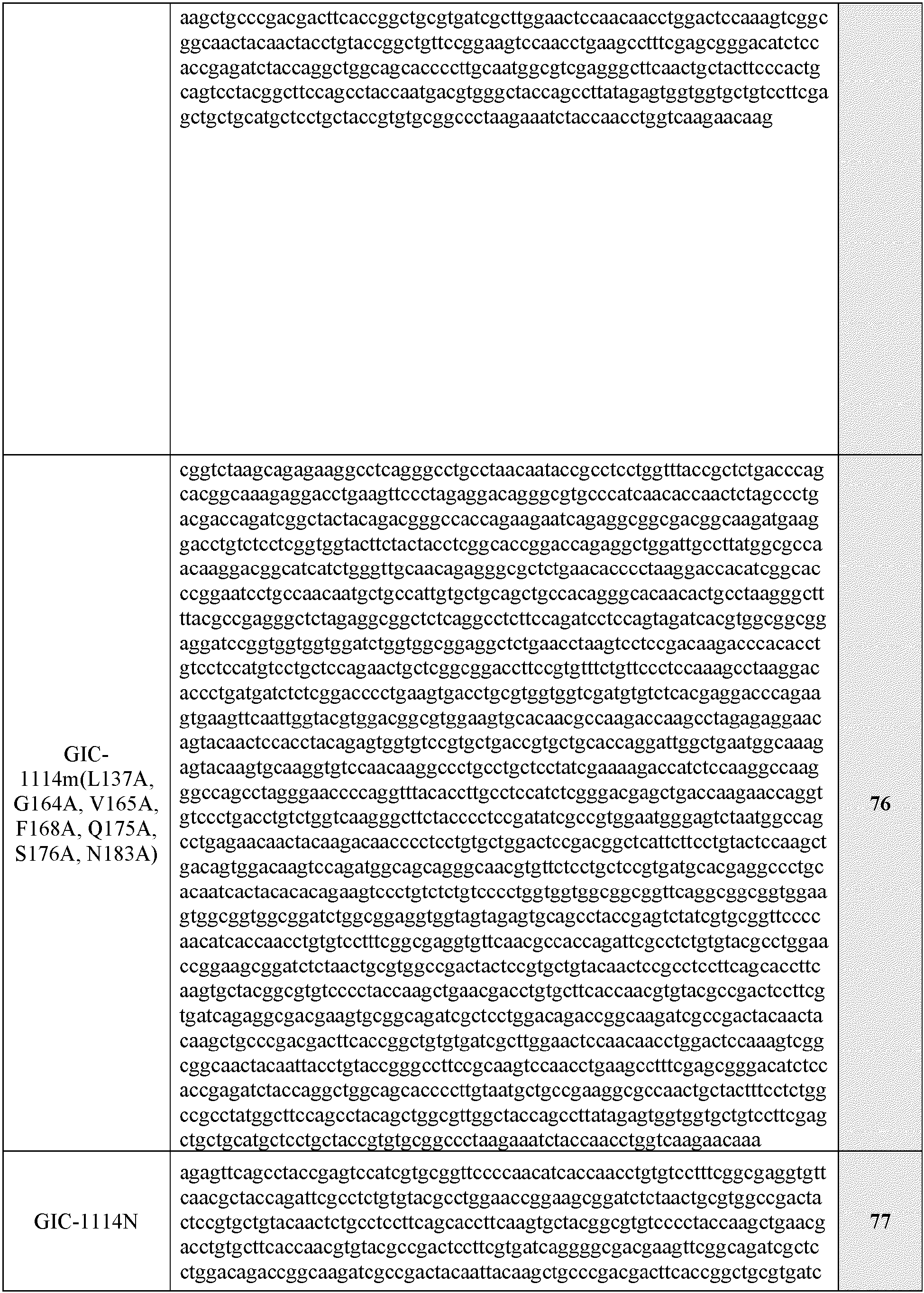

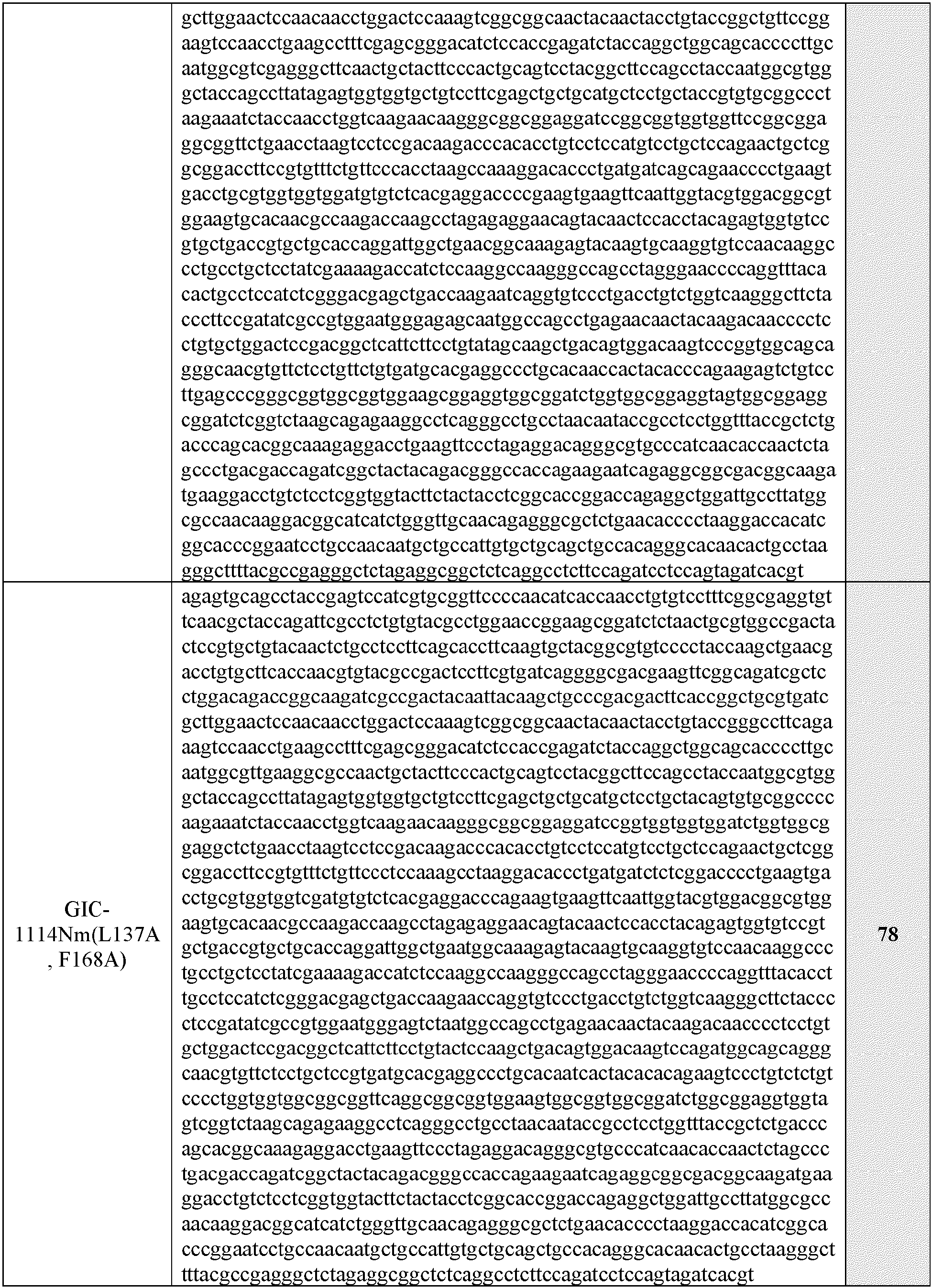

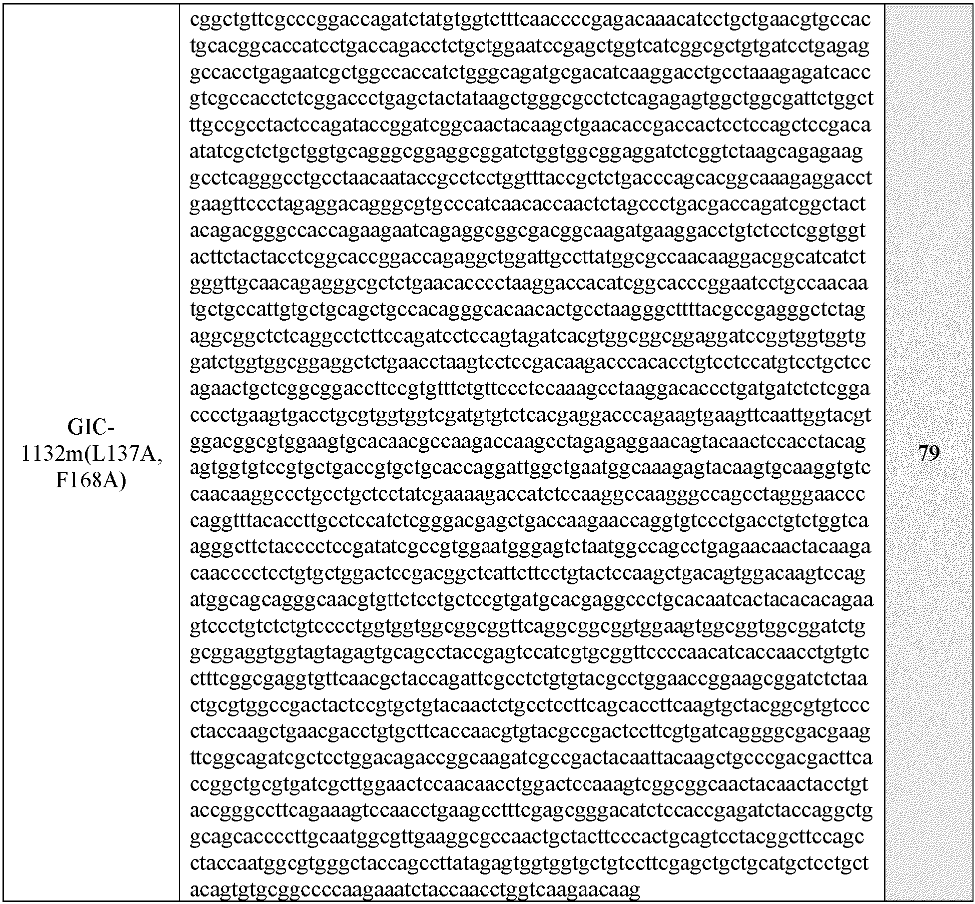

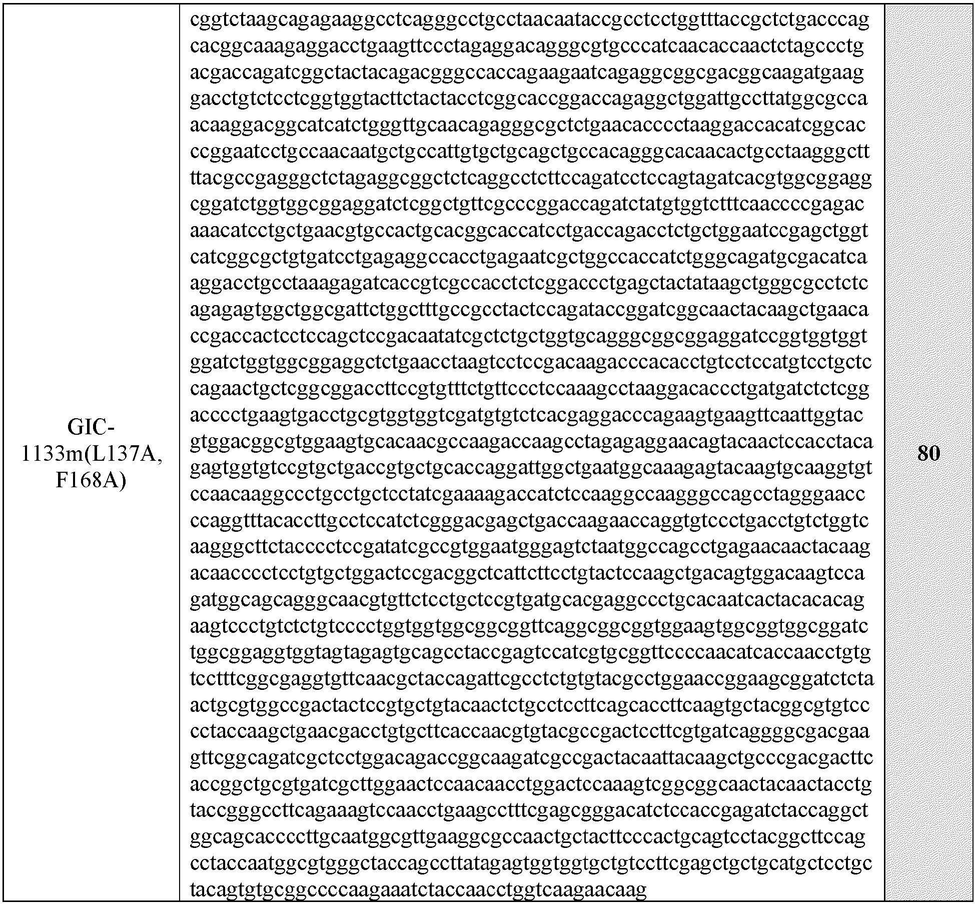

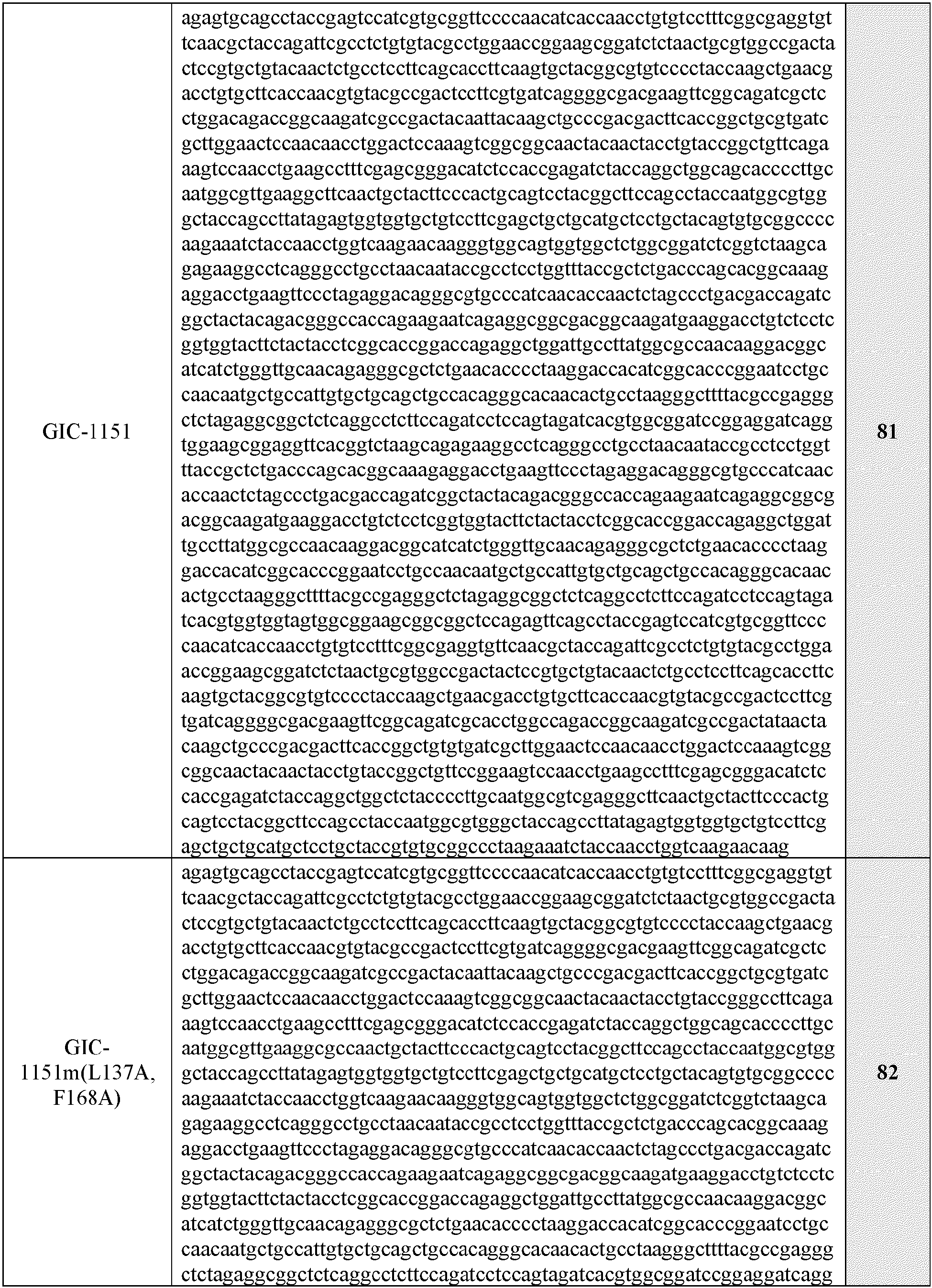

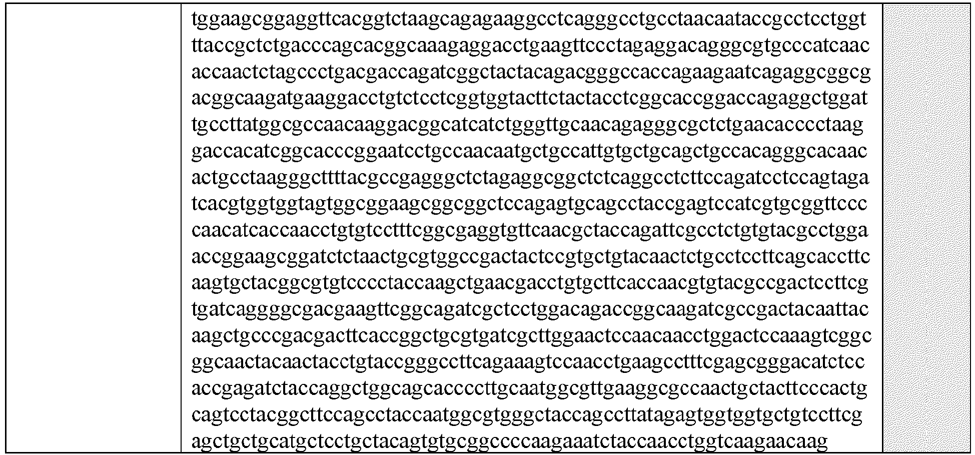

### Example 2: Assessment of binding affinity between fusion protein and ACE2

AHC biosensor (Anti-hIgG Fc capture, ForteBio, 18-5060) was hydrated in advance in a 96-well microplate (Greiner Bio-One, Cat: 655209), each well thereof containing 200 ul of 1X kinetic buffer (10X Kinetics buffer, ForteBio, 18-1042). The ligand (fusion protein) to be attached to the biosensor was diluted to a concentration of 10 ug/ml in 1X Ni-NTA kinetic buffer. The hACE2 (His-Tag, Aero, AC2-H52H8) to be attached to the ligand was diluted in 1X kinetic buffer to a concentration of 500 nM, 250 nM, 125 nM, 62.5 nM, 31.2 nM, 15.6 nM, 7.8 nM or 60 nM (single concentration). 200 µl of each reagent was added to each well of a 96-well microplate (Greiner Bio-One, Cat: 655209), and the program was set.

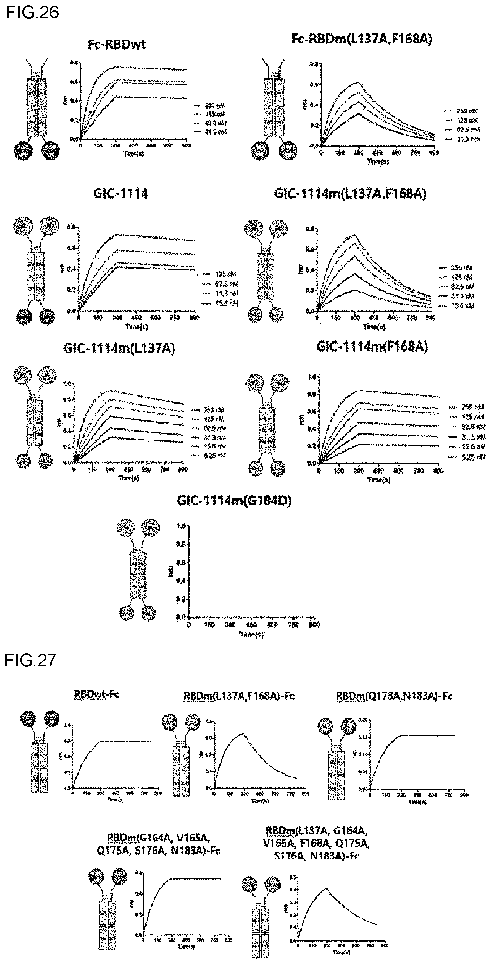

The binding affinity to ACE2 of the fusion protein comprising wild-type RBD (RBDwt) or variant RBD (RBDm) linked to the C-terminus of the Fc domain, among the fusion proteins prepared in Example 1, was detected (confirmation at each receptor concentration). As shown in FIG. 26, the result showed that the fusion protein comprising wild-type RBD exhibited high binding affinity (low K_{D} value) for the ACE2 receptor, whereas a fusion protein comprising the RBD variant (RBDm (L137A, F168A)) exhibited low binding affinity (high K_{D} value) for the ACE2 receptor. Meanwhile, the fusion protein comprising the RBD variant having either the L137A or the F168A mutation alone showed a low degree of dissociation after binding, but the fusion protein comprising variant RBD having both L137A and F168A mutations exhibited a high dissociation rate after binding (FIG. 26). The RBD variant (RBDm (G184D)) having a single mutation of G184D did not bind to ACE2 at all. The binding affinity of each fusion protein is shown in Table 5 below.

**[Table 5]**

| Binding affinity for ACE2 of fusion protein | | | | |
|---|---|---|---|---|
| | K_{D} (M) | Kₒₙ(1/Ms) | K_{dis}(1/s) | Full R^2 |
| Fc-RBDwt | 1.32E-09 | 4.65E+04 | 6.12E-05 | 0.9943 |
| Fc-RBDm(L 13 7A,F 168A) | 1.05E-07 | 2.69E+04 | 2.82E-03 | 0.9806 |
| GIC-1114 | 1.93E-09 | 6.60E+04 | 1.27E-04 | 0.9924 |
| GIC-1114m(L137A,F168A) | 1.17E-07 | 2.39E+04 | 2.79E-03 | 0.9906 |
| GIC-1114m(L137A) | 9.23E-09 | 3.79E+04 | 3.50E-04 | 0.9915 |
| GIC-1114m(F168A) | 4.86E-09 | 3.27E+04 | 1.59E-04 | 0.9901 |
| GIC-1114m(G184D) | ND | ND | ND | ND |

In addition, the binding affinity for ACE2 of the fusion protein comprising wild-type RBD (RBDwt) or variant RBD (RBDm) linked to the N-terminus of the Fc domain, among the fusion proteins prepared in Example 1, was detected (single concentration). As shown in FIG. 27, the fusion protein comprising the wild-type RBD or variant RBD exhibited the same results regardless of the linking position of the N-terminus or C-terminus of the Fc domain. These results mean that the RBD variant itself in which a specific amino acid has been mutated affects the binding affinity for ACE2, regardless of the binding position of the Fc domain or RBD. In addition, in the case where further amino acid substitutions (G164A, V165A, F168A, Q175A, S176A, N183A) were present, in addition to L137A and F168A, the binding affinity was decreased, but in the case where L137A and F168A were not present (G164A, V165A, F168A, Q175A, S176A and N183A variants), binding affinity was maintained.

Overall, it was found that the binding force of the fusion protein comprising the RBD variant of the present invention was reduced by 10 to 100 times or more compared to the fusion protein comprising wild-type RBD, and the results mean that when i) L137A and F168A mutations, or ii) G184D mutation is present, the binding affinity for ACE2 receptor of RBD is remarkably reduced.

### Example 3: Confirmation of hACE2 binding efficiency and hACE2 downregulation of fusion protein

HEK293T cells expressing hACE2 (HEK293T/hACE2, Genecopoeia, Cat No: #SL221) were sufficiently grown and were seeded at a concentration of 3×10⁵ cells/100 µl FACS buffer (5% (v/v) FBS in PBS) in each FACS tube. The proteins shown in Table 6 below were mixed in each tube, and were then allowed to react at 4°C for 1 hour.

**[Table 6]**

| **product** | **Amounts** |
|---|---|
| Fc control (IgG1) | 0.4 ug/ml |
| GIC-1114 | 1 ug/ml |
| GIC-1114m(L137A,F168A) | 1 ug/ml |
| GIC-1114m(G184D) | 1 ug/ml |

Then, each sample was centrifuged at 4°C at 300xg for 5 minutes to remove the supernatant, and the pellet was suspended in 1 ml of FACS buffer. After repeating the centrifugation and suspension processes twice, anti-human IgG (H+L) antibody (FITC; Invitrogen, CAT No: #31259) was diluted to 1:500 with a FACS buffer, and the sample was treated with 100 µl of the dilution and reacted at 4°C for 30 minutes. Then, each sample was centrifuged at 4°C at 300xg for 5 minutes to remove the supernatant, and the pellet was suspended in 1 ml of FACS buffer. After the centrifugation and suspension processes were repeated twice to remove the unbound antibody, each sample was suspended in 500 µl of FACS buffer, and the FITC intensity was measured using a flow cytometer (BD FACSCelesta^{™}, BD, CA, USA) to determine the binding efficiency of each fusion protein to hACE2.

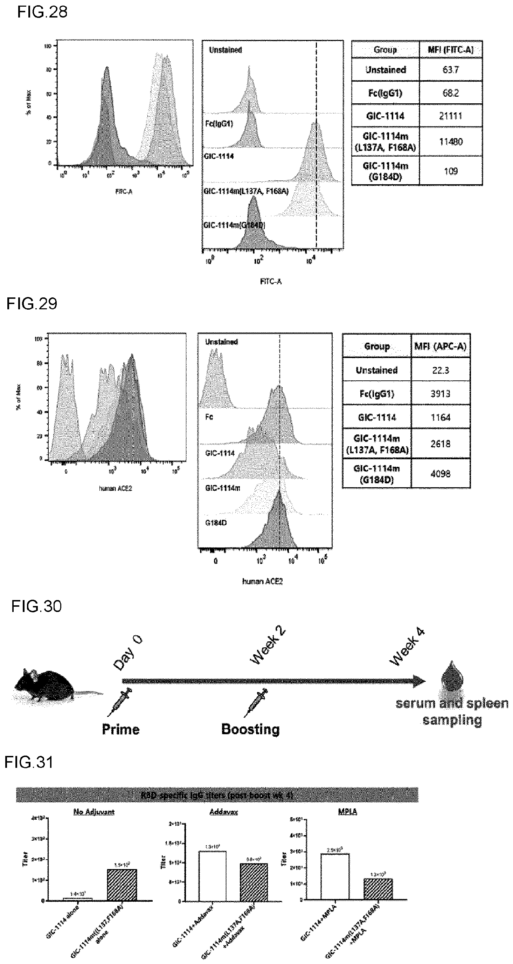

As shown in FIG. 28, the result showed that the fusion proteins (GIC-1114m (L137A, F168A) and GIC-1114m (G184D)) comprising the RBD variant exhibit remarkably lower hACE2 binding efficiency than the fusion protein comprising wild-type RBD (GIC-1114).

Whether or not the identified reduced binding efficiency of the fusion protein comprising each of the receptor-binding domain variants leads to inhibition of hACE2 expression downregulation was determined. Specifically, HEK293T cells expressing hACE2 (HEK293T/hACE2, Genecopoeia, Cat No: #SL221) were sufficiently grown and were seeded at a concentration of 3×10⁵ cells/100 µl FACS buffer (5% (v/v) FBS in PBS) in each FACS tube. The proteins shown in Table 6 below were mixed in each tube and were then allowed to react at 37°C for 1 hour. Then, each sample was centrifuged at 4°C at 300xg for 5 minutes to remove the supernatant, and the pellet was suspended in 1 ml of FACS buffer. After repeating the centrifugation and suspension processes twice, goat anti-human ACE-2 ectodomain antibody (R&D system, AF933) was diluted to a final concentration of 2 ug/ml with a FACS buffer, and the sample was treated with 100 µl of the dilution and allowed to react at 4°C for 1 hour. Then, each sample was centrifuged at 4°C at 300xg for 5 minutes to remove the supernatant, and the pellet was suspended in 1 ml of FACS buffer. After the centrifugation and suspension processes were repeated twice to remove the unbound antibody, Rabbit anti-goat IgG(H+L) cross-adsorbed secondary antibody (DyLight 650; Invitrogen, SA5-10081) was diluted to 1:1000 with FACS buffer, and the sample was treated with 100 µl of the dilution and allowed to react at 4°C for 30 minutes. Then, each sample was centrifuged at 4°C at 300xg for 5 minutes to remove the supernatant, and the pellet was suspended in 1 ml of FACS buffer. After the centrifugation and suspension processes were repeated twice, each sample was suspended in 500 µl of FACS buffer and the FITC intensity was measured using a flow cytometer (BD FACSCelesta^{™}, BD, CA, USA) to determine the decreased (downregulated) expression of hACE2.

As can be seen from FIG. 29, treatment with the fusion protein (GIC-1114) comprising wild-type RBD downregulated the expression of hACE2, but treatment with the fusion protein comprising the RBD variants (GIC-1114m (L137A, F168A) and GIC-1114m (G184D)) exhibited a remarkably reduced effect of hACE2 expression in HEK293.

Therefore, unlike wild-type RBD, the RBD variant of the present invention exhibits low binding affinity and binding efficiency for hACE2, and does not cause a decrease in the expression of hACE2, thus being useful as a vaccine for coronavirus since the risk of side effects of conventional vaccines due to decreased expression of hACE2 by wild-type RBD is expected to be very low, even when used as an immunogen (antigen) for vaccines.

### Example 4: Assessment of immune response upon vaccination of mice with fusion protein of present invention

### Example 4-1: Preparation and vaccination of mice

The mice and the fusion protein used in animal experiments for evaluating the immunogenicity of the fusion protein produced in Example 1 are shown in Table 7 below.

**[Table 7]**

| Animal information | | Strain of origin | | Manufacturer | | month | | Sex | |
|---|---|---|---|---|---|---|---|---|---|
| mouse | | C57BL/6 | | ORIENT BIO Inc. | | 6 weeks old | | Female | |

| Sub jects information | | | Manufacturer | | Cat # | | Volume | | Amounts |
|---|---|---|---|---|---|---|---|---|---|
| Subjects | GIC-1114 | | GI-Cell | | N/A | | 50 µl | | 20 µg |
| | GIC-1114m(L137A,F168A) | | GI-Cell | | N/A | | 50 µl | | 20 µg |
| | GIC-1151m(L137A,F168A) | | GI-Cell | | N/A | | 50 µl | | 12 µg |
| Adjuvant | Addavax | | Invivogen | | vac-adx-10 | | 50 µl | | - |
| | MPLA | | Invivogen | | vac-mpla | | 50 µl | | 5 µg |

The dosage was controlled to have the same number of moles in consideration of the molecular weight of each fusion protein (GIC-1114(N-Fc-RBDwt): 140kDa, GIC-1114m(N-Fc-RBDmt_L137A,F168A): 140kDa, GIC-1151m(RBDmt_L137A,F168A-N-N-RBDmt_L137A,F168A): 85kDa).

The fusion protein was administered by intramuscular injection through the femoral muscle, and two weeks after the primary administration, the fusion protein was further administered. 4 weeks after the administration, blood was collected through the abdominal vein of each subject, and the serum was separated and stored in a cryogenic freezer. In addition, the spleen of each subject was removed at the 4^{th} week and mixed with RPMI1640 medium + 10% FBS, and the resulting mixture was filled in a 15 mL conical tube to prepare a serum in an ice-cold state (FIG. 30).

### Example 4-2: Assessment of neutralizing antibodies formation

ELISA was performed to confirm the titer of mouse neutralizing antibody formation in the serum collected in Example 4-1. Specifically, SARS-CoV-2 (COVID-19) S protein RBD (ACRO biosystem, SPD-C53H3) or human recombinant IgG1 protein (Sinobiologics, 10702-HNAH) was diluted in 1X PBS (10X PBS (Welgene, ML 008-02) 1:10 dilution) and was loaded at 100 µl (500 ng/well) in a 96-well plate (BioLegend, 423501), covered with an adhesive plastic, and incubated at 4°C overnight.

A 1X ELISA assay diluent (5X ELISA Assay diluent (BioLegend, 421203), 1X PBS dilution) was added in an amount of 100 µL to each well of the plate and allowed to stand at room temperature for 2 hours while shaking at 100 rpm. The supernatant was removed from each plate, and was washed twice with 200 µL of washing buffer (0.05% of Tween20 (Sigma, P7949) in 1X PBS). 254 µL of 1X ELISA assay diluent was added to 2 µL of serum collected from each mouse to perform primary dilution(1:128). Then, serial dilution to 1:2 1:4, 1:8, 1:16, 1:32, 1:64 and 1:128 was further performed, and 100 µL of the diluted serum was added to each well, and then allowed to stand at room temperature for 2 hours while shaking at 100 rpm. The diluted sample was removed and washed 3 times with 200 µL of washing buffer. 100 µL of a goat anti-mouse IgG (H+L) Ab (Invitrogen, 31430) diluent (1X ELISA Assay diluent, 1:10000) was added to each well and incubated in the dark at room temperature for 90 minutes while shaking at 100 rpm. After the supernatant was removed, the residue was washed 5 times with 200 µL of washing buffer. 100 µL of TMB solution (BioLegend, 421101) was added to each well, the reaction was allowed to proceed at room temperature for 1 minute, and 100 µL of a stop solution (BioLegend, 77316) was added to each well to stop the reaction. The OD value of each sample was measured at 450 nm using an ELISA reader (Molecular device, SpectraMax iD3), and the titer of the neutralizing antibody formation was detected.

As can be seen from FIG. 31, in the case where vaccination is performed using a fusion protein without adjuvant, when a fusion protein comprising variant RBD (GIC-1114m(L137A, F168A)) was administered, a remarkably high titer of the RBD-specific neutralizing antibody was obtained compared to when a fusion protein (GIC-1114) comprising wild-type RBD was administered.

In addition, when the adjuvant was administered in combination to boost the immune response, each antibody titer was improved. When GIC-1114 or GIC-1114m (L137A, F168A) was used, the neutralizing antibody titer was similar.

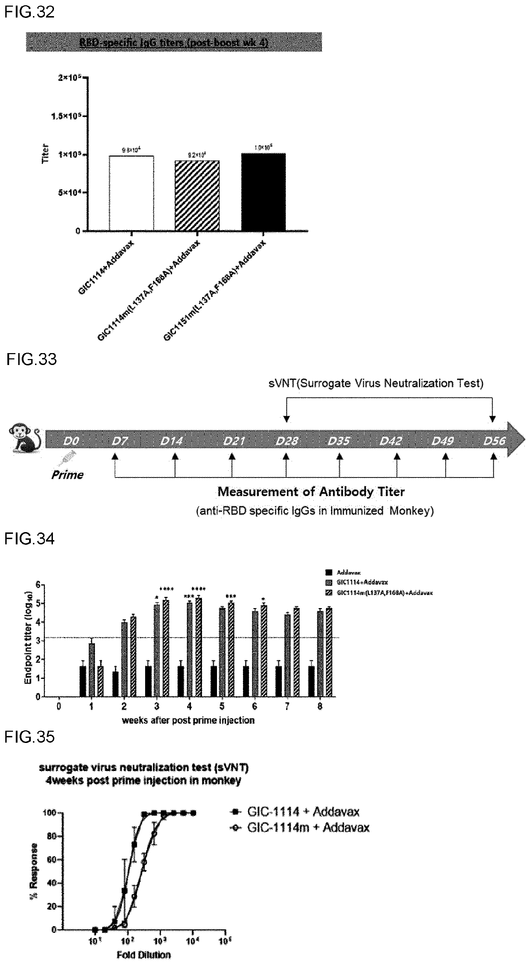

In the case of GIC-1151m (L137A, F168A), which are fusion proteins not comprising an Fc domain, the SARS-CoV2 RBD-specific neutralizing antibody titer of the serum obtained 4 weeks after inoculation to mice with Addavax as an adjuvant was determined. As shown in FIG. 32, GIC-1151m (L137A, F168A) exhibited similar levels of RBD-specific neutralizing antibody titers to GIC-1114 and GIC-1114m (L137A, F168A).

The above results show that the variant RBD and the fusion protein comprising the same do not affect the titer of the SARS-CoV2 RBD-specific antibody despite the genetic mutation and the decreased ACE2 binding affinity, thus exhibiting immunogenicity comparable or superior to that of a vaccine composition comprising wild-type RBD, while reducing side effects.

### Example 5: Assessment of immune response upon vaccination of monkey model with fusion protein comprising RBD variant

### Example 5-1: Preparation and vaccination of monkeys

The monkeys and the fusion protein used in animal experiments for evaluating the immunogenicity of the fusion protein produced in Example 1 are shown in Table 8 below.

**[Table 8]**

| Animal information | | Strain of origin | Manufacturer | month | Sex |
|---|---|---|---|---|---|
| monkey | | Cynomolgus | ORIENT CAM | 39-47 months old | Mate |

| Subject information | | Manufacturer | Cat # | Volume | Amounts |
|---|---|---|---|---|---|
| Subject | GIC-1114 | GI-Cell | N/A | 250 µl | 25 µg |
| | GIC-1114m (L137A,F168A) | GI-Cell | N/A | 250 µl | 25 µg |
| Adjuvant | Addavax | Invivogen | vac-adx-10 | 250 µl | - |

The fusion protein was administered to a monkey model in a constant amount of 25 µg by intramuscular injection through the femoral muscle, and blood was collected from the femoral vein at 7-day intervals after inoculation (8 times over 8 weeks) to measure the titer of the antibody, and the SARS-CoV-2 surrogate virus neutralization test (sVNT) was performed 28 and 56 days after inoculation.

The collected blood was transferred to an SST tube, allowed to stand at room temperature for 30 minutes, and centrifuged at 4°C and 3,000 rpm for 10 minutes to obtain serum, which was stored in a 1.7 mL microcentrifuge tube (Axygen, USA) (FIG. 33).

### Example 5-2: Assessment of neutralizing antibody titer

The neutralizing antibody titer contained in the serum collected from the monkey model was detected using the same method as described in Example 4-2. As shown in FIG. 34, GIC-1114 showed a high titer of the RBD-specific neutralizing antibody formation at one week after inoculation, but did not reach a reference titer (2880) indicating a significant vaccine capability according to USFDA criteria, and exceeded the reference titer level after 2 weeks, and the blood of monkeys to which GIC-1114m (L137A, F168A) comprising the RBD variant was administered exhibited a higher titer of the neutralizing antibody formation than monkeys to which GIC-1114 comprising wild-type RBD was administered. The titer of the antibody was the highest between about 3 weeks to 4 weeks, and the high antibody titer was maintained without a significant decrease until 8 weeks, which demonstrates that, even when the RBD variant and fusion protein of the present invention were administered only once, long-term immunity can be obtained without additional immune boosting.

### Example 5-3: SARS-CoV-2 surrogate virus neutralization test (sVNT) of fusion protein

A surrogate virus neutralization test (sVNT) was performed in order to determine whether or not the antibody of the blood collected from the monkey model inoculated with the fusion protein of the present invention can effectively neutralize SARS-CoV-2.

Specifically, ELISA was performed using an ACE2: SARS-CoV-2 spike inhibitor screening assay kit (BPS Bioscience, Cat#79936). ACE2-His (Cat#11003) in the assay kit was diluted in PBS to a final concentration of 1 µg/ml, and 50 µl of the diluted solution was seeded into a well plate and attached thereto for 1 hour. After attachment, the supernatant was removed, and each plate was washed three times with 100 µl of 1X Immuno Buffer 1 and blocked with 100 µl of Blocking Buffer 2 for 10 minutes, after which the supernatant was removed.

10 µl of a serum sample dilution from the monkey model (final dilution to 1:64) and 20 µl of 1X Immuno Buffer 1 were seeded into each well of the prepared plate, and an inhibitor buffer (PBS) was seeded at 10 pl/well for a positive control and blank. 20 µl of SARS-CoV-2 Spike (diluted to a final concentration of 1 ng/ul) was seeded in the wells marked as Positive Control and Test Inhibitor, and 20 µl of the inhibitor buffer (PBS) was seeded in the well marked as Blank, and was then reacted at room temperature for 1 hour. Each plate was washed three times with 100 µl of 1X Immuno Buffer 1, and was then blocked with 100 µl of Blocking Buffer 2 for 10 minutes. Secondary HRP-labeled antibody 1 (Cat#52130H) was diluted to 1000X using Blocking Buffer 2, and was then seeded at 100 pl/well and reacted at room temperature for 1 hour. Each plate was washed three times with 100 µl of 1X Immuno Buffer 1, and was then blocked with 100 µl of Blocking Buffer 2 for 10 minutes. ELISA ECL Substrate A and ELISA ECL Substrate B were mixed at a ratio of 1:1, the resulting mixture was seeded at 100 pl/well, and the luminescence value of the sample was measured using a GloMax^{®} Discover Microplate Reader (Promega).

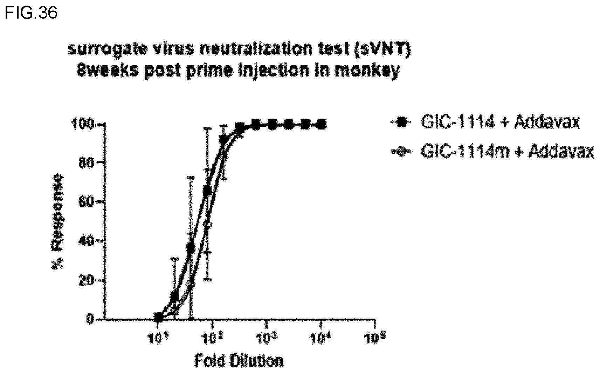

As shown in FIGS. 35 and 36, the results of an sVNT test of serum collected from monkey models showed that SARS-CoV-2 antibody comprised in the serum of monkeys administered with GIC-1114m (L137A, F168A) comprising RBD variants, among the fusion proteins of the present invention, exhibited remarkably higher SARS-CoV-2 neutralizing ability than that of monkeys administered with GIC-1114 comprising wild-type RBD.

Overall, the above results demonstrated that an antibody having a high titer and neutralizing ability can be formed upon administration with the RBD variant of the present invention and the fusion protein comprising the same.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### Industrial Applicability

The coronavirus-derived receptor-binding domain variant of the present invention has remarkably reduced binding force to ACE2, and thus can overcome the drawbacks of conventional vaccines using the coronavirus spike protein or receptor-binding domain thereof, wherein the reduced ACE2 expression due to binding to ACE2 and negative feedback may lead to side effects of the lungs or heart, and in particular, may be fatal to patients suffering from underlying diseases of the lungs or heart. In particular, the fusion protein constructed by combining the coronavirus receptor-binding domain with the Fc domain of the present invention is imparted with a greatly improved in-vivo half-life, and has superior efficacy by further combining a coronavirus-derived domain, for example, N protein, M protein, ORF protein, or the like, therewith through additional modification, thus being highly applicable to a multivalent immunogenic composition. Therefore, the coronavirus receptor-binding domain variant of the present invention is useful for the prevention and treatment of coronavirus infections comprising COVID-19.

## Claims

1. A coronavirus-derived receptor-binding domain variant having reduced binding affinity to an ACE2 receptor.

2. The coronavirus-derived receptor-binding domain variant according to claim 1, wherein the receptor-binding domain variant comprises a mutation at at least one position selected from the group consisting of L137, G164, V165, F168, Q175, S176, N183 and G184 in an amino acid sequence of SEQ ID NO: 1.

3. The coronavirus-derived receptor-binding domain variant according to claim 2, wherein the receptor-binding domain variant comprises at least one mutation selected from the group consisting of: a mutation of amino acids L137 and F168; and a mutation of amino acid G184.

4. The coronavirus-derived receptor-binding domain variant according to claim 2, wherein the mutation is substitution.

5. The coronavirus-derived receptor-binding domain variant according to claim 4, wherein the receptor-binding domain variant comprises at least one substitution selected from the group consisting of: a substitution of amino acids L137A and F168A; and a substitution of amino acid G184D.

6. The coronavirus-derived receptor-binding domain variant according to claim 1, wherein the coronavirus is SARS-CoV-2.

7. A fusion protein comprising the receptor-binding domain variant according to any one of claims 1 to 6.

8. The fusion protein according to claim 7, further comprising a coronavirus-derived substance.

9. The fusion protein according to claim 8, wherein the coronavirus-derived substance comprises at least one selected from the group consisting of SARS-CoV-2-derived N proteins and M proteins.

10. The fusion protein according to claim 8, wherein the receptor-binding domain variant and the SARS-CoV-2-derived substance are linked by a glycine-serine linker.

11. The fusion protein according to claim 7, further comprising an Fc domain.

12. The fusion protein according to claim 11, wherein the receptor-binding domain variant is linked to at least one of an N-terminus and a C-terminus of the Fc domain.

13. The fusion protein according to claim 12, wherein the receptor-binding domain variant is linked to one of the N-terminus and the C-terminus of the Fc domain, and at least one coronavirus-derived substance is linked to a remaining one thereof.

14. The fusion protein according to claim 11, wherein the Fc domain is an Fc domain of immunoglobulin G (IgG).

15. The fusion protein according to claim 11, wherein the fusion protein is a dimer of a protein comprising a receptor-binding domain variant and an Fc domain.

16. The fusion protein according to claim 15, wherein the fusion protein is a dimer of a protein further comprising at least one coronavirus-derived substance.

17. A vaccine composition comprising:
the receptor-binding domain variant according to any one of claims 1 to 6; or
a fusion protein comprising the receptor-binding domain variant.

18. The vaccine composition according to claim 17, further comprising an adjuvant.

19. The vaccine composition according to claim 18, wherein the adjuvant comprises at least one selected from the group consisting of aluminum salts, squalene, squalene-containing emulsions, calcium salts, dsRNA analogues, lipopolysaccharides, lipid A analogues, flagellins, imidazoquinolines, CpG ODN, mineral oils, Toll-like receptor (TLR) antagonists, C-type lectin ligands, CD1d ligands, detergents, liposomes, saponins, cytokines and peptides.

20. A method for vaccination against coronavirus infection, the method comprising administering the vaccine composition according to any one of claims 17 to 19 to a subject.

21. A pharmaceutical composition for preventing or treating coronavirus infection comprising the receptor-binding domain variant according to any one of claims 1 to 6, or a fusion protein comprising the receptor-binding domain variant as an active ingredient.

22. The pharmaceutical composition according to claim 21, wherein the coronavirus infection is COVID-19.

23. A method for preventing or treating coronavirus infection, the method comprising administering the pharmaceutical composition according to claim 21 to a subject.

24. A nucleic acid encoding the receptor-binding domain variant according to any one of claims 1 to 6, or a fusion protein comprising the receptor-binding domain variant.

25. A recombinant vector comprising the nucleic acid according to claim 24.

26. A host cell into which the nucleic acid according to claim 24, or the recombinant vector according to claim 25 is introduced.
